# EUROPEAN PATENT APPLICATION

(11) **EP 4 403 572 A1**
(43) Date of publication of application: **24.07.2024**
(21) Application number: 22866787.9
(22) Date of filing: 13.09.2022
(51) Int. Cl.: C07K 16/28, C12N 15/13, C12N 15/63, C12N 5/10, A61K 39/395, A61P 35/00

(54) **ANTI-HUMAN CD3 ANTIBODY AND USE THEREOF**

(30) Priority: 13.09.2021 CN 202111069892
(71) Applicant: Shandong Simcere Biopharmaceutical Co., Ltd., Shandong 264006 (CN)
(72) Inventor: WANG, Qiong, Shanghai 201318 (CN); FU, Yayuan, Shanghai 201318 (CN); WEI, Peipei, Shanghai 201318 (CN); CAO, Zhuoxiao, Shanghai 201318 (CN); TANG, Renhong, Shanghai 201318 (CN); REN, Jinsheng, Nanjing, Jiangsu 210042 (CN)
(74) Representative: Richly & Ritschel Patentanwälte PartG mbB
(86) International application number: PCT/CN2022/118334
(87) International publication number: WO 2023/036326

(57) **Abstract**

Provided are an anti-human CD3 antibody and the use thereof. Specifically, provided are an antibody specifically binding to human CD3 or an antigen binding fragment thereof, a multi-specific antigen binding molecule, an isolated nucleic acid molecule, a vector, a cell and an immune effector cell thereof, a method for preparing the antibody or the antigen binding fragment thereof, the multi-specific antigen binding molecule and the immune effector cell, a pharmaceutical composition, the pharmaceutical use and a method for treating diseases.

## Description

### Cross Reference to Related Application

The present application claims the right of priority for the invention patent application filed to the China National Intellectual Property Administration on September 13, 2021, with the title of invention "Anti-human CD3 antibody and the use thereof' and the filling number of CN 202111069892.7, the entire contents of which are incorporated herein by reference in its entirety.

### Technical Field

The present application relates to the field of biomedicine, and specifically to an anti-human CD3 antibody and the use thereof.

### Background

T cell receptor (TCR) is a specific receptor on the surface of T cells, with two subunits, TCRαβ and TCRγδ, whose main function is to recognize and bind to antigens presented by MHC molecules. The cytoplasmic region of TCR is very short, and TCR alone cannot transmit signals, requiring the help of CD3 molecules. CD3 molecule is an important differentiation antigen on T cell membrane, mainly consisting of four chains, namely ε chain, δ chain, γ chain and ζ chain. In a CD3-TCR complex, CD3γ, CD3δ and CD3ε are present in two non-covalent bond forms of CD3γε and CD3δε heterodimers, with the ζ chain mostly being present in the form of a homodimer, i.e., ζ-ζ. The cytoplasmic domains of CD3ε, CD3δ, CD3γ and CD3ζ all contain a conserved immune receptor tyrosine-based activation motif (ITAM). CD3ζ has three ITAMs, while the other CD3 chains each contain one ITAM. Different antigen stimuli can induce different phosphorylation of the 10 ITAMs in TCR, ultimately triggering different downstream signaling pathways. The cytoplasmic domain of CD3ε has a basic residue-rich sequence (BRS) in the membrane-proximal region, followed by a non-redundant proline residue-rich sequence (PRS) and an ITAM. Therefore, BRS in the CD3ε subunit can effectively recruit Lck through ionic interaction to initiate TCR phosphorylation. It has been shown that certain antibodies against CD3ε can induce TCR signaling, while the other antibodies cannot. On the basis of the important role of CD3 in TCR signaling and immune response, it is of great significance to develop antibodies against CD3.

In addition, multi-specific antibodies, by binding to target antigens on tumor cells and CD3 on T cells, form TCR-independent artificial immune synapses and circumvent the restriction of HLA. However, CD3 antibodies are at risk of developing cytokine storms. Faroudi *et al.* observed that the activation threshold for target cell lysis was more than 1000 times more sensitive than that for cytokine release. This difference was mainly caused by the difference in the concentration of a target cell surface antigen and the difference in the number of pMHC:TCR complexes. Low affinity facilitates consecutive triggering of many TCRs by some peptide-MHC complexes through constant rapid binding and dissociation. This consecutive triggering is critical to maintain signal transmission within a certain period of time, which allows T cell to eventually reach the activation threshold. While anti-CD3 antibodies having high affinity may be difficult to dissociate, which fails to consecutively trigger TCR, so as not to effectively stimulate T cells, indicating that antibodies having a suitable affinity range may be more effective in stimulating T cells via TCR signaling.

Therefore, there is still a great demand for developing novel anti-CD3 antibodies.

### Summary of the Invention

The present application provides an antibody specifically binding to human CD3 or an antigen binding fragment thereof, a multi-specific antigen binding molecule, an isolated nucleic acid molecule, a vector, a cell and an immune effector cell thereof, a method for preparing the antibody or the antigen binding fragment thereof, the multi-specific antigen binding molecule and the immune effector cell, a pharmaceutical composition, the pharmaceutical use and a method for treating diseases.

In a first aspect, the present application provides an antibody specifically binding to human CD3 or an antigen binding fragment thereof, wherein the antibody or the antigen binding fragment thereof comprises a heavy chain variable region and/or a light chain variable region, the heavy chain variable region comprises HCDR1-3, the light chain variable region comprises LCDR1-3, and the HCDR1-3 and/or the LCDR1-3 are selected from Table 10 or Table 18.

In some embodiments of the first aspect, the HCDR1 comprises a sequence as shown in any one of SEQ ID NOs: 31-33, 44-46, 57-59, 70-72, 83-85, 96-98 and 109-111, or a sequence having at least 70% identity or at most 3 amino acid mutations or differences compared thereto; and
the HCDR2 comprises a sequence as shown in any one of SEQ ID NOs: 34-36, 137, 47-49, 60-62, 73-75, 86-88, 99-101 and 112-114, or a sequence having at least 70% identity or at most 3 amino acid mutations or differences compared thereto; and
the HCDR3 comprises a sequence as shown in any one of SEQ ID NOs: 37-38, 138-139, 50-51, 142-143, 63-64, 76-77, 89-90, 102-103 and 115-116, or a sequence having at least 70% identity or at most 3 amino acid mutations or differences compared thereto; and/or
the LCDR1 comprises a sequence as shown in any one of SEQ ID NOs: 39-40, 140-141, 52-53, 65-66, 78-79, 91-92, 104-105 and 117-118, or a sequence having at least 70% identity or at most 3 amino acid mutations or differences compared thereto; and
the LCDR2 comprises a sequence as shown in any one of SEQ ID NOs: 41-42, 54-55, 67-68, 80-81, 93-94, 106-107 and 119-120, or a sequence having at least 70% identity or at most 3 amino acid mutations or differences compared thereto; and
the LCDR3 comprises a sequence as shown in any one of SEQ ID NOs: 43, 56, 69, 82, 95, 108 and 121, or a sequence having at least 70% identity or at most 3 amino acid mutations or differences compared thereto.

In some embodiments of the first aspect, the HCDR1-3 and/or the LCDR1-3 are determined according to the Kabat, Chothia or IMGT method.

In some embodiments of the first aspect, the heavy chain variable region comprises a sequence as shown in any one of SEQ ID NOs: 17, 19, 21, 23, 25, 27, 29, 124, 126, 128, 130, 132, 134 and 136, or a sequence having at least 70% identity or at most 15 amino acid mutations or differences compared thereto.

In some embodiments of the first aspect, the light chain variable region comprises a sequence as shown in any one of SEQ ID NOs: 18, 20, 22, 24, 26, 28, 30, 123, 125, 127, 129, 131, 133 and 135, or a sequence having at least 70% identity or at most 15 amino acid mutations or differences compared thereto.

In some embodiments of the first aspect, the at least 70% identity is at least 75%, at least 80%, at least 85%, at least 90%, at least 91%, at least 92%, at least 93%, at least 94%, at least 95%, at least 96%, at least 97%, at least 98%, at least 99% or 100% identity.

In some embodiments of the first aspect, the at most 3 mutations or differences are at most 3, 2, 1 or 0 mutations or differences.

In some embodiments of the first aspect, the at most 15 mutations or differences are at most 15, 14, 13, 12, 11, 10, 9, 8, 7, 6, 5, 4, 3, 2, 1 or 0 mutations or differences.

In some embodiments of the first aspect, the mutation is insertion, deletion or substitution. In some embodiments, the substitution is a conservative amino acid substitution.

In some embodiments of the first aspect, the mutation is a back mutation or a hotspot mutation.

In some embodiments of the first aspect, the heavy chain variable region of the antibody or the antigen binding fragment thereof has an amino acid sequence as shown in SEQ ID NO: 17, and the light chain variable region of the antibody or the antigen binding fragment thereof has an amino acid sequence as shown in SEQ ID NO: 18; or
the heavy chain variable region of the antibody or the antigen binding fragment thereof has an amino acid sequence as shown in SEQ ID NO: 19, and the light chain variable region of the antibody or the antigen binding fragment thereof has an amino acid sequence as shown in SEQ ID NO: 20; or
the heavy chain variable region of the antibody or the antigen binding fragment thereof has an amino acid sequence as shown in SEQ ID NO: 21, and the light chain variable region of the antibody or the antigen binding fragment thereof has an amino acid sequence as shown in SEQ ID NO: 22; or
the heavy chain variable region of the antibody or the antigen binding fragment thereof has an amino acid sequence as shown in SEQ ID NO: 23, and the light chain variable region of the antibody or the antigen binding fragment thereof has an amino acid sequence as shown in SEQ ID NO: 24; or
the heavy chain variable region of the antibody or the antigen binding fragment thereof has an amino acid sequence as shown in SEQ ID NO: 25, and the light chain variable region of the antibody or the antigen binding fragment thereof has an amino acid sequence as shown in SEQ ID NO: 26; or
the heavy chain variable region of the antibody or the antigen binding fragment thereof has an amino acid sequence as shown in SEQ ID NO: 27, and the light chain variable region of the antibody or the antigen binding fragment thereof has an amino acid sequence as shown in SEQ ID NO: 28; or
the heavy chain variable region of the antibody or the antigen binding fragment thereof has an amino acid sequence as shown in SEQ ID NO: 29, and the light chain variable region of the antibody or the antigen binding fragment thereof has an amino acid sequence as shown in SEQ ID NO: 30; or
the heavy chain variable region of the antibody or the antigen binding fragment thereof has an amino acid sequence as shown in SEQ ID NO: 124, and the light chain variable region of the antibody or the antigen binding fragment thereof has an amino acid sequence as shown in SEQ ID NO: 123; or
the heavy chain variable region of the antibody or the antigen binding fragment thereof has an amino acid sequence as shown in SEQ ID NO: 126, and the light chain variable region of the antibody or the antigen binding fragment thereof has an amino acid sequence as shown in SEQ ID NO: 125; or
the heavy chain variable region of the antibody or the antigen binding fragment thereof has an amino acid sequence as shown in SEQ ID NO: 128, and the light chain variable region of the antibody or the antigen binding fragment thereof has an amino acid sequence as shown in SEQ ID NO: 127; or
the heavy chain variable region of the antibody or the antigen binding fragment thereof has an amino acid sequence as shown in SEQ ID NO: 130, and the light chain variable region of the antibody or the antigen binding fragment thereof has an amino acid sequence as shown in SEQ ID NO: 129; or
the heavy chain variable region of the antibody or the antigen binding fragment thereof has an amino acid sequence as shown in SEQ ID NO: 132, and the light chain variable region of the antibody or the antigen binding fragment thereof has an amino acid sequence as shown in SEQ ID NO: 131; or
the heavy chain variable region of the antibody or the antigen binding fragment thereof has an amino acid sequence as shown in SEQ ID NO: 134, and the light chain variable region of the antibody or the antigen binding fragment thereof has an amino acid sequence as shown in SEQ ID NO: 133; or
the heavy chain variable region of the antibody or the antigen binding fragment thereof has an amino acid sequence as shown in SEQ ID NO: 136, and the light chain variable region of the antibody or the antigen binding fragment thereof has an amino acid sequence as shown in SEQ ID NO: 135.

In some embodiments of the first aspect, the antibody or the antigen binding fragment thereof further comprises a heavy chain constant region and/or a light chain constant region.

In some embodiments of the first aspect, the heavy chain constant region is selected from IgG, such as IgG1, IgG2, IgG3 or IgG4.

In some embodiments of the first aspect, the heavy chain constant region is selected from human IgG, such as human IgG1, human IgG2, human IgG3 or human IgG4.

In some particular embodiments of the first aspect, the heavy chain constant region can be selected from SEQ ID NO: 13.

In some embodiments of the first aspect, the light chain constant region is selected from a κ chain or a λ chain.

In some particular embodiments of the first aspect, the light chain constant region can be selected from SEQ ID NO: 14 and SEQ ID NO: 122.

In some embodiments of the first aspect, the antibody or the antigen binding fragment thereof is selected from: a monoclonal antibody, a polyclonal antibody, a natural antibody, an engineered antibody, a mono-specific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, a fully human antibody, Fab, Fab', Fab'-SH, F(ab')₂, Fd, Fv, scFv, a diabody and a single domain antibody.

In some embodiments of the first aspect, the antibody or the antigen binding fragment thereof is conjugated with a therapeutic agent or a tracer.

In some embodiments of the first aspect, the therapeutic agent can be selected from: a radioactive isotope, a chemotherapeutic drug and an immunomodulator.

In some embodiments of the first aspect, the tracer can be selected from: a radiological contrast agent, a paramagnetic ion, a metal, a fluorescent tag, a chemiluminescence tag, an ultrasound contrast agent and a photosensitizer.

In some embodiments of the first aspect, the antibody or the antigen binding fragment thereof also binds to monkey CD3.

In some embodiments of the first aspect, the CD3 is selected from a CD3ε subunit and a dimer comprising a CD3ε subunit, such as a CD3ε/δ dimer.

In a second aspect, the present application provides a multi-specific antigen binding molecule, wherein the multi-specific antigen binding molecule comprises at least a first antigen binding module and a second antigen binding module, the first antigen binding module comprises the antibody or the antigen binding fragment thereof of the first aspect, and/or the second antigen binding module binds to other targets different from the first antigen binding module or binds to different epitopes of the same target.

In some embodiments of the second aspect, the other targets are selected from a tumor specific antigen (TSA) and a tumor associated antigen (TAA).

In some embodiments of the second aspect, the multi-specific antigen binding molecule is a bispecific T cell engager (BiTE).

In a third aspect, the present application provides an isolated nucleic acid molecule, wherein the nucleic acid molecule encodes the antibody or the antigen binding fragment thereof of the first aspect, or the multi-specific antigen binding molecule of the second aspect.

In some embodiments of the third aspect, the nucleic acid molecule also encodes a chimeric antigen receptor.

In some embodiments of the third aspect, the nucleic acid encoding the antibody or the antigen binding fragment thereof of the first aspect, or the multi-specific antigen binding molecule of the second aspect, is linked to the nucleic acid encoding the chimeric antigen receptor via a nucleic acid sequence encoding a self-cleaving peptide or an IRES nucleic acid sequence.

In a fourth aspect, the present application provides a vector, which comprises the nucleic acid molecule of the third aspect.

In a fifth aspect, the present application provides a cell, which comprises the vector of the fourth aspect.

In a sixth aspect, the present application provides an immune effector cell, which expresses a chimeric antigen receptor, and/or expresses the antibody or the antigen binding fragment thereof of the first aspect, or the multi-specific antigen binding molecule of the second aspect.

In some embodiments of the sixth aspect, the immune effector cell is selected from: a T cell, a natural killer cell (NK cell), a natural killer T cell (NKT cell), a monocyte, a macrophage, a dendritic cell and a mast cell.

In some embodiments of the sixth aspect, the T cell is selected from: a cytotoxic T cell, a regulatory T cell and a helper T cell.

In some embodiments of the sixth aspect, the immune effector cell is an autologous immune effector cell or an allogeneic immune effector cell.

In a seventh aspect, the present application provides a method for preparing the antibody or the antigen binding fragment thereof of the first aspect, or the multi-specific antigen binding molecule of the second aspect, wherein the method comprises: (1) culturing the cell of the fifth aspect; and/or (2) isolating the antibody or the antigen binding fragment thereof, or the multi-specific antigen binding molecule expressed by the cell.

In an eighth aspect, the present application provides a method for preparing the immune effector cell of the sixth aspect, wherein the method comprises: (1) introducing into a naive immune effector cell a. a nucleic acid molecule encoding the chimeric antigen receptor and b. a nucleic acid molecule encoding the antibody or the antigen binding fragment thereof of the first aspect, or the multi-specific antigen binding molecule of the second aspect; and/or (2) making the immune effector cell into which the above-mentioned nucleic acid molecules are introduced express the chimeric antigen receptor, and the antibody or the antigen binding fragment thereof of the first aspect, or the multi-specific antigen binding molecule of the second aspect.

In a ninth aspect, the present application provides a pharmaceutical composition, wherein the pharmaceutical composition comprises the antibody or the antigen binding fragment thereof of the first aspect, the multi-specific antigen binding molecule of the second aspect, the nucleic acid molecule of the third aspect, the vector of the fourth aspect, the cell of the fifth aspect, the immune effector cell of the sixth aspect or a product prepared according to the method of the seventh or eighth aspect.

In some embodiments of the ninth aspect, the composition further comprises a pharmaceutically acceptable carrier, diluent or adjuvant.

In a tenth aspect, the present application provides the use of the antibody or the antigen binding fragment thereof of the first aspect, the multi-specific antigen binding molecule of the second aspect, the nucleic acid molecule of the third aspect, the vector of the fourth aspect, the cell of the fifth aspect, the immune effector cell of the sixth aspect, a product prepared according to the method of the seventh or eighth aspect, or the pharmaceutical composition of the ninth aspect in the preparation of a drug for treating a tumor or a cancer.

In some embodiments of the tenth aspect, the tumor or the cancer is selected from a hematological tumor and a solid tumor.

In some embodiments of the tenth aspect, the hematological tumor can be selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma.

In some embodiments of the tenth aspect, the solid tumor can be selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer and liver cancer.

In an eleventh aspect, the present application provides a method for treating a tumor or a cancer, wherein the method comprises administering to a subject an effective amount of a drug, and the drug comprises the antibody or the antigen binding fragment thereof of the first aspect, the multi-specific antigen binding molecule of the second aspect, the nucleic acid molecule of the third aspect, the vector of the fourth aspect, the cell of the fifth aspect, the immune effector cell of the sixth aspect, a product prepared according to the method of the seventh or eighth aspect, or the pharmaceutical composition of the ninth aspect.

In some embodiments of the eleventh aspect, the tumor or the cancer is selected from a hematological tumor and a solid tumor.

In some embodiments of the eleventh aspect, the hematological tumor can be selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma.

In some embodiments of the eleventh aspect, the solid tumor can be selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer and liver cancer.

In a twelfth aspect, the present application provides the antibody or the antigen binding fragment thereof of the first aspect, the multi-specific antigen binding molecule of the second aspect, the nucleic acid molecule of the third aspect, the vector of the fourth aspect, the cell of the fifth aspect, the immune effector cell of the sixth aspect, a product prepared according to the method of the seventh or eighth aspect, or the pharmaceutical composition of the ninth aspect, for use in the treatment of a tumor or a cancer.

In some embodiments of the twelfth aspect, the tumor or the cancer is selected from a hematological tumor and a solid tumor.

In some embodiments of the twelfth aspect, the hematological tumor can be selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma.

In some embodiments of the twelfth aspect, the solid tumor can be selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer or liver cancer.

The embodiments of any one of the first to twelfth aspects of the present application have at least one or more of the following beneficial effects:
(1) A novel anti-CD3 antibody that is different from the existing antibodies and is screened by the method of the present application specifically binds to a CD3ε subunit or a complex thereof, or specifically binds to a cell, especially a T cell, expressing the CD3ε subunit or the complex thereof;
(2) The antibody of the present disclosure has an appropriate affinity for CD3, can effectively stimulate T cells via TCR signaling, preferably an antibody that efficiently activates T cells is obtained.

### Definition and Description of Terminology

Unless defined otherwise in the present application, scientific and technical terms related to the present application shall have the meanings understood by one of ordinary skill in the art.

In addition, unless otherwise specified herein, terms in the singular form herein shall include that in the plural form, and terms in the plural form shall include that in the singular form. More specifically, as used in the description and the appended claims, the singular forms "a/an" and "this" include plural referents, unless otherwise clearly stated.

The terms "include", "comprise" and "have" are used interchangeably herein and are intended to indicate the inclusiveness of the solution, meaning that the solution can have other elements than those listed. Furthermore, it should be understood that the description of "include", "comprise" and "have" as used herein also provides the solution of "consist of". Illustratively, "a composition comprising A and B" should be understood as the following technical solution: composition consisting of A and B, and composition containing other components in addition to A and B, fall within the scope of the aforementioned "a composition".

The term "and/or" as used herein includes the meanings of "and", "or" and "all or any other combination of elements linked by the term".

The term "specifically bind" herein means that an antigen binding molecule (e.g., an antibody) typically specifically binds to an antigen and substantially the same antigen with a high affinity, but does not bind to an unrelated antigen with a high affinity. Affinity is typically reflected by the equilibrium dissociation constant (KD), where lower KD indicates higher affinity. Taking an antibody as an example, a high affinity typically means having a KD of about 10⁻⁶ M or less, about 10⁻⁷ M or less, about 10⁻⁸ M or less, about 1 × 10⁻⁹ M or less, about 1 × 10⁻¹⁰ M or less, about 1 × 10⁻¹¹ M or less, or about 1 × 10⁻¹² M or less. KD is calculated as follows: KD = Kd/Ka, where Kd represents the dissociation rate and Ka represents the binding rate. The equilibrium dissociation constant KD can be measured using well-known methods in the art, such as surface plasmon resonance (e.g., Biacore) or equilibrium dialysis assay. Exemplarily, see the method for obtaining the KD value as shown in example 8 herein.

The term "antigen binding molecule" is used herein in the broadest sense and refers to a molecule specifically binding to an antigen. Exemplarily, the antigen binding molecule includes, but is not limited to, an antibody or an antibody mimetic. The "antibody mimetic" refers to an organic compound or a binding domain that can specifically bind to an antigen, but is not structurally related to an antibody. Exemplarily, the antibody mimetic includes, but is not limited to, affibody, affitin, affilin, a designed ankyrin repeat protein (DARPin), a nucleic acid aptamer or a Kunitz-type domain peptide.

The term "antibody" is used herein in the broadest sense and refers to a polypeptide or a combination of polypeptides comprising sufficient sequences from a heavy chain variable region of an immunoglobulin and/or sufficient sequences from a light chain variable region of an immunoglobulin to be able to specifically bind to an antigen. The "antibody" herein encompasses various forms and various structures as long as they exhibit the desired antigen-binding activity. The "antibody" herein includes an alternative protein scaffold or artificial scaffold with grafted complementarity determining regions (CDRs) or CDR derivatives. Such scaffolds include antibody-derived scaffolds comprising mutations introduced, e.g., to stabilize the three-dimensional structure of the antibody, and fully synthetic scaffolds comprising, e.g., biocompatible polymers. See, for example, Korndorfer et al., 2003, Proteins: Structure, Function, and Bioinformatics, 53(1): 121-129 (2003); Roque et al., Biotechnol. Prog. 20:639-654 (2004). Such scaffolds may also include non-antibody-derived scaffolds, for example, scaffold proteins known in the art which can be used for grafting CDRs, including but not limited to tenascin, fibronectin, a peptide aptamer, etc.

The "antibody" herein includes a typical "four-chain antibody", which belongs to an immunoglobulin consisting of two heavy chains (HC) and two light chains (LC); the heavy chain refers to a polypeptide chain that consists of, in a direction from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is an IgE isotype, it optionally also comprises a heavy chain constant region CH4 domain; the light chain is a polypeptide chain consisting of, in a direction from the N-terminus to the C-terminus, a light chain variable region (VL) and a light chain constant region (CL); the heavy chain is linked to the heavy chain and the heavy chain is linked to the light chain via disulfide bonds, forming a Y-shaped structure. Since the amino acid composition and arrangement sequence of the heavy chain constant region of an immunoglobulin are different, the antigenicity of the immunoglobulin is also different. Accordingly, "immunoglobulin" herein can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3 and IgG4, and IgA can be divided into IgA1 and IgA2. The light chains are divided into a κ chain or a λ chain by the difference in the constant region. Each Ig class of the five Ig classes can have either a κ chain or a λ chain.

The "antibody" herein also includes an antibody that does not comprise light chains, for example, heavy-chain antibodies (HCAbs) produced from Camelus dromedarius, Camelus bactrianus, Lama glama, Lama guanicoe and Vicugna pacos, and Ig new antigen receptors (IgNARs) found in Chondrichthyes such as sharks.

The "antibody" herein may be derived from any animal, including but not limited to humans and non-human animals selected from primates, mammals, rodents and vertebrates, such as Camelidae, Lama glama, Lama guanicoe, Vicugna pacos, sheep, rabbits, mice, rats or Chondrichthyes (such as sharks).

The "antibody" herein includes but is not limited to a monoclonal antibody, a polyclonal antibody, a mono-specific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, or a fully human antibody.

The term "monoclonal antibody" herein refers to an antibody obtained from a population of substantially homogeneous antibodies, that is, individual antibodies comprising the population are identical and/or bind to the same epitope, except for possible variants (such as those containing naturally occurring mutations or produced during the production of a preparation, such variants generally being present in a small amount). In contrast to polyclonal antibody preparations that typically include different antibodies targeting different determinants (epitopes), each monoclonal antibody in monoclonal antibody preparations targets a single determinant on an antigen. The modifier "monoclonal" herein should not be construed as requiring the production of the antibody or the antigen binding molecule thereof by any particular method. For example, monoclonal antibodies can be prepared by a variety of techniques, including (but not limited to) hybridoma techniques, recombinant DNA methods, phage library display techniques, methods using transgenic animals containing all or part of human immunoglobulin loci, and other methods known in the art.

The term "natural antibody" herein refers to an antibody produced and paired by the immune system of a multicellular organism. The antibody of the term "engineered antibody" herein refers to a non-natural antibody obtained by techniques such as genetic engineering and antibody engineering. Exemplarily, the "engineered antibody" includes a humanized antibody, a small molecule antibody (such as scFv), a bispecific antibody, etc.

The term "monospecific" herein refers to having one or more binding sites, wherein each binding site binds to the same epitope of the same antigen.

The term "multi-specific antibody" herein refers to an antibody having at least two antigen binding sites, each of which binds to a different epitope of the same antigen or to a different epitope of a different antigen. Therefore, terms such as "bispecific", "trispecific" and "tetraspecific" refer to the number of different epitopes to which an antibody/antigen binding molecule can bind.

The term "valence" herein refers to the presence of a defined number of binding sites in an antibody/an antigen binding molecule. Therefore, the terms "monovalent", "bivalent", "tetravalent" and "hexavalent" indicate the presence of one binding site, two binding sites, four binding sites and six binding sites in an antibody/antigen binding molecule, respectively.

The "full-length antibody", "complete antibody" and "intact antibody" are used interchangeably herein and mean that they have a structure substantially similar to that of a natural antibody.

The "antigen binding fragment" and "antibody fragment" are used interchangeably herein, which do not possess the full structure of an intact antibody, and only comprise a part of an intact antibody or a variant of the part, wherein the part of the intact antibody or the variant of the part has the ability to bind to an antigen. The "antigen binding fragment" or "antibody fragment" herein includes but is not limited to Fab, Fab', Fab'-SH, F(ab')₂, Fd, Fv, scFv, a diabody and a single domain antibody.

An intact antibody is digested with papain to produce two identical antigen binding fragments, referred to as "Fab" fragments, each containing a variable domain of heavy chain and a variable domain of light chain, and also a constant domain of the light chain and the first constant domain (CH1) of the heavy chain. Thus, the term "Fab fragment" herein refers to an antibody fragment comprising the VL domain and the constant domain (CL) of a light chain, and the VH domain and the first constant domain (CH1) of a heavy chain. The Fab' fragment differs from the Fab fragment by adding a small number of residues at the carboxyl terminus of the CH1 domain of the heavy chain, including one or more cysteines from the hinge region of the antibody. Fab'-SH is a Fab' fragment in which the cysteine residue of the constant domain carries a free thiol group. Treatment with pepsin produces a F(ab')₂ fragment having two antigen binding sites (two Fab fragments) and a portion of the Fc region.

The term "Fd" herein refers to an antibody consisting of VH and CH1 domains. The term "Fv" herein refers to an antibody fragment consisting of a single arm VL and a VH domain. The Fv fragment is generally considered to be the smallest antibody fragment that can form an intact antigen binding site. It is generally believed that the six CDRs confer antigen binding specificity to an antibody. However, even one variable region (such as a Fd fragment, which contains only three antigen-specific CDRs) is able to recognize and bind to an antigen, although it may have a lower affinity than an intact binding site.

The term "scFv" (single-chain variable fragment) herein refers to a single polypeptide chain comprising a VL domain and a VH domain, wherein the VL and the VH are linked via a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Roseburg and Moore eds., Springer-Verlag, New York, pages 269-315 (1994)). Such scFv molecules can have a general structure: NH2-VL-linker-VH-COOH or NH2-VH-linker-VL-COOH. Suitable linkers in the prior art consist of repeated GGGGS amino acid sequences or variants thereof. For example, linkers having an amino acid sequence (GGGGS)₄ can be used, but variants thereof can also be used (Holliger et al., (1993), Proc. Natl. Acad. Sci. USA 90: 6444-6448). Other linkers that can be used in the present application are described by Alfthan et al., (1995), Protein Eng. 8: 725-731, Choi et al., (2001), Eur. J. Immunol. 31: 94-106, Hu et al., (1996), Cancer Res. 56: 3055-3061, Kipriyanov et al., (1999), J. Mol. Biol. 293: 41-56 and Roovers et al., (2001), Cancer Immunol. In some cases, a disulfide bond can also be present between VH and VL of scFv, forming a disulfide-linked Fv (dsFv).

The VH and VL domains of the term "diabody" herein are expressed on a single polypeptide chain, but a linker used is too short to allow for pairing between two domains of the same chain, thereby forcing the domain to pair with the complementary domain of the other chain and producing two antigen binding sites (see, for example, Holliger P. et al., Proc. Natl. Acad. Sci. USA 90: 6444-6448 (1993), and Poljak R.J. et al., Structure 2: 1121-1123 (1994)).

The terms "single domain antibody (sdAb)", "VHH" and "nanobody" herein have the same meaning and are used interchangeably, and refer to a single domain antibody consisting of only one heavy chain variable region constructed by cloning the heavy chain variable region of an antibody, which is the smallest antigen binding fragment with full functionality. Typically, a single domain antibody consisting of only one heavy chain variable region is constructed by obtaining an antibody that naturally lacks light chains and heavy chain constant region 1 (CH1), and then cloning the heavy chain variable region of the antibody. Single domain antibody can be derived from a Camelidae heavy chain antibody or Chondrichthyes fish IgNAR.

The term "naked antibody" herein refers to an antibody that is not conjugated with a therapeutic agent or a tracer; and the term "conjugated antibody" herein refers to an antibody that is conjugated with a therapeutic agent or a tracer.

The term "chimeric antibody" herein refers to an antibody in which a portion of the light chain or/and heavy chain thereof is derived from one antibody (which may be derived from a particular species or belong to a particular antibody class or subclass), and another portion of the light chain or/and the heavy chain is derived from another antibody (which may be derived from the same or a different species or belong to the same or a different antibody class or subclass), but which in any case retains the activity of binding to an antigen of interest (U.S.P 4,816,567, Cabilly et al.; Morrison et al., Proc. Natl. Acad. Sci. USA, 81: 6851-6855 (1984)). For example, the term "chimeric antibody" may include an antibody (e.g., a human-murine chimeric antibody) in which the heavy and light chain variable regions of the antibody are derived from a first antibody (e.g., a murine-derived antibody) and the heavy and light chain constant regions of the antibody are derived from a second antibody (e.g., a human antibody).

The term "humanized antibody" herein refers to a genetically engineered non-human antibody whose amino acid sequence has been modified to increase sequence homology with a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody generally retains or partially retains the expected properties of a donor antibody, including but not limited to antigen specificity, affinity, reactivity, ability to improve immune cell activities, ability to enhance immune responses, etc.

The term "fully human antibody" herein refers to an antibody having variable regions in which both the FRs and CDRs are derived from human germline immunoglobulin sequences. Furthermore, if the antibody comprises a constant region, the constant region also is derived from human germline immunoglobulin sequences. The "fully human antibody" herein may include amino acid residues not encoded by human germline immunoglobulin sequences (for example, mutations introduced by random or site-specific mutagenesis in vitro or by somatic mutation in vivo). However, the "fully human antibody" herein does not include an antibody in which CDR sequences derived from the germline of another mammalian species (e.g., a mouse) have been grafted onto human framework sequences.

The term "variable region" herein refers to a region in the heavy or light chain of an antibody that is involved in enabling the antibody to bind to an antigen. "Heavy chain variable region", "VH" and "HCVR" are used interchangeably, and "light chain variable region", "VL" and "LCVR" are used interchangeably. The variable domains of the heavy and light chains (VH and VL, respectively) of a natural antibody generally have similar structures, and each domain comprises four conserved framework regions (FRs) and three hypervariable regions (HVRs). See, for example, Kindt et al., Kuby Immunology, 6th ed., W.H.Freeman and Co., p. 91 (2007). A single VH or VL domain may be sufficient to confer antigen binding specificity. The terms "complementarity determining region" and "CDR" are used interchangeably herein, and usually refer to the hypervariable region (HVR) of either the heavy chain variable region (VH) or the light chain variable region (VL), which is also referred to as the complementarity determining region because it can form precise complementarity with an antigenic epitope in the spatial structure, wherein the heavy chain variable region CDR can be abbreviated as HCDR and the light chain variable region CDR can be abbreviated as LCDR. The term "framework region" or "FR region" is interchangeable and refers to those amino acid residues other than CDRs in the heavy chain variable region or light chain variable region of an antibody. Usually, a typical antibody variable region consists of 4 FR regions and 3 CDR regions in the following order: FR1-CDR1-FR2-CDR2-FR3-CDR3-FR4.

For a further description of CDR, with reference to Kabat et al., J. Biol. Chem., 252: 6609-6616 (1977); Kabat et al., United States Department of Health and Human Services, "Sequences of proteins of immunological interest" (1991); Chothia et al., J. Mol. Biol. 196: 901-917 (1987); Al-Lazikani B. et al., J. Mol. Biol., 273: 927-948 (1997); MacCallum et al., J. Mol. Biol. 262: 732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309: 657-670 (2001). The "CDR" can be defined by the Kabat numbering system. Tool websites include abYsis website (www.abysis.org/abysis/sequence input/key_ annotation/key_annotation.cgi)

The term "Kabat numbering system" herein generally refers to the immunoglobulin alignment and numbering system proposed by Elvin A. Kabat (see, for example, Kabat et al., Sequences of Proteins of Immunological Interest, 5th Ed., Public Health Service, National Institutes of Health, Bethesda, Md., 1991).

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" comprises at least: a CH1 domain, a hinge region, a CH2 domain, a CH3 domain, or variants or fragments thereof. The "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" can be selected from CH1, Fc or CH3 domains.

The term "light chain constant region" herein refers to the carboxyl terminus portion of an antibody light chain, which is not directly involved in the binding of an antibody to an antigen, and the light chain constant region can be selected from a constant κ domain or a constant λ domain.

The term "Fc" herein refers to the carboxyl terminus portion of an intact antibody obtained by hydrolyzing the antibody with papain, which typically comprises CH3 and CH2 domains of an antibody. The Fc region includes, for example, an Fc region of a natural sequence, a recombinant Fc region and a variant Fc region. Although the boundary of the Fc region of an immunoglobulin heavy chain can be slightly changed, the Fc region of a human IgG heavy chain is usually defined as the region extending from the amino acid residue at position Cys226 or from Pro230 to its carboxyl terminus. The C-terminal lysine of the Fc region (residue 447 according to the Kabat numbering system) can be removed, for example, during the production or purification of an antibody, or by recombinant engineering of a nucleic acid encoding an antibody heavy chain, so the Fc region may or may not include Lys447.

The term "conservative amino acid" herein generally refers to amino acids that belong to the same class or have similar characteristics (such as charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:

Exemplarily, the following six groups are examples of amino acids that are considered to be conservative substitutions for one another:
1) Alanine (A), Serine (S) and Threonine (T);
2) Aspartic acid (D) and Glutamic acid (E);
3) Asparagine (N) and Glutamine (Q);
4) Arginine (R), Lysine (K) and Histidine (H);
5) Isoleucine (I), Leucine (L), Methionine (M) and Valine (V); and
6) Phenylalanine (F), Tyrosine (Y) and Tryptophan (W).

The term "identity" herein can be calculated by the following method: to determine the percent "identity" of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The percent identity between two sequences will vary with the identical positions shared by the sequences, considering the number of gaps that need to be introduced to optimally align the two sequences and the length of each gap.

The sequence comparison and the calculation of percent identity between two sequences can be achieved using a mathematical algorithm. For example, the percent identity between two amino acid sequences is determined using the Needlema and Wunsch algorithm ((1970) J. Mol. Biol. 48: 444-453) in the GAP program that has been integrated into the GCG software package (available at www.gcg.com) and using the Blossum 62 matrix or the PAM250 matrix, with a gap weight of 16, 14, 12, 10, 8, 6 or 4 and a length weight of 1, 2, 3, 4, 5 or 6. For another example, the percent identity between two nucleotide sequences is determined using the GAP program in the GCG software package (available at www.gcg.com) and using the NWSgapdna.CMP matrix, with a gap weight of 40, 50, 60, 70, or 80 and a length weight of 1, 2, 3, 4, 5, or 6. A particularly preferred parameter set (and one parameter set that should be used unless otherwise stated) is the Blossum62 scoring matrix with a gap penalty of 12, a gap extension penalty of 4, and a gap frameshift penalty of 5.

The percent identity between two amino acid sequences or nucleotide sequences can also be determined using the PAM120 weighted remainder table, with a gap length penalty of 12 and a gap penalty of 4, and using the E. Meyers and W. Miller algorithm ((1989) CABIOS, 4: 11-17) that has been incorporated into the ALIGN program (version 2.0).

Optionally, the nucleic acid and protein sequences of the present application can further be used as "query sequences" to perform searches against public databases, e.g., to identify other family member sequences or related sequences. For example, the NBLAST and XBLAST programs (version 2.0) of Altschul et al., (1990) J. Mol. Biol. 215: 403-10 can be used to perform such searches. BLAST nucleotide searches can be performed with the NBLAST program, with score = 100 and word length = 12, to obtain the nucleotide sequences homologous to the nucleic acid molecule (SEQ ID NO: 1) of the present application. BLAST protein searches can be performed with the XBLAST program, with score = 50 and word length = 3, to obtain the amino acid sequences homologous to the protein molecule of the present application. To obtain gapped alignment results for comparison purposes, gapped BLAST can be used as described in Altschul et al., (1997) Nucleic Acids Res. 25: 3389-3402. When BLAST and gapped BLAST programs are used, the default parameters of the corresponding programs (e.g., XBLAST and NBLAST) can be used. See www.ncbi.nlm.nih.gov.

The term "chimeric antigen receptor (CAR)" herein refers to an artificial cell surface receptor engineered to express on immune effector cells and specifically bind to an antigen, comprising at least (1) an extracellular antigen binding domain, such as a variable heavy chain or light chain of an antibody, (2) a transmembrane domain anchoring the CAR into an immune effector cell, and (3) an intracellular signaling domain. The CAR can redirect T cells and other immune effector cells to selected targets, such as cancer cells, in a non-MHC-restricted way by using an extracellular antigen-binding domain.

The term "nucleic acid" herein includes any compound and/or substance comprising a polymer of nucleotides. Each nucleotide consists of a base, specifically a purine or pyrimidine base (i.e., cytosine (C), guanine (G), adenine (A), thymine (T) or uracil (U)), a sugar (i.e., deoxyribose or ribose) and a phosphate group. Generally, a nucleic acid molecule is described by a sequence of bases, whereby the bases represent the primary structure (linear structure) of the nucleic acid molecule. The sequence of bases is usually expressed as 5' to 3'. In this context, the term nucleic acid molecule encompasses deoxyribonucleic acid (DNA), including for example complementary DNA (cDNA) and genomic DNA, ribonucleic acid (RNA), especially messenger RNA (mRNA), synthetic forms of DNA or RNA, and a polymer comprising a mixture of two or more of these molecules. The nucleic acid molecule can be linear or circular. Furthermore, the term nucleic acid molecule includes both sense strand and antisense strand, as well as single-stranded form and double-stranded form. Furthermore, the nucleic acid molecules described herein may contain naturally occurring or non-naturally occurring nucleotides. Examples of non-naturally occurring nucleotides include modified nucleotide bases with derivatized sugar or phosphate backbone linkages or chemically modified residues. The nucleic acid molecule also encompasses DNA and RNA molecules which are suitable as vectors for direct expression of the antibody of the present application *in vitro* and/or *in vivo,* for example in a host or patient. Such DNA (e.g., cDNA) or RNA (e.g., mRNA) vectors may be unmodified or modified. For example, mRNA can be chemically modified to enhance the stability of the RNA vector and/or the expression of the encoded molecule, so that the mRNA can be injected into a subject to generate an antibody in vivo (see, for example, Stadler et al., Nature Medicine 2017, Published online June 12, 2017, doi: 10.1038/nm.4356 or EP 2 101 823 B1). The "isolated" nucleic acid herein refers to a nucleic acid molecule that has been separated from components of the natural environment thereof. The isolated nucleic acid includes a nucleic acid molecule contained in a cell that normally contains the nucleic acid molecule, but which is present extrachromosomally or at a chromosomal location other than the natural chromosomal location thereof.

The term "vector" herein refers to a nucleic acid molecule capable of amplifying another nucleic acid to which it is linked. The term includes a vector having a self-replicating nucleic acid structure and a vector that is integrated into the genome of a host cell into which the vector has been introduced. Certain vectors can direct the expression of nucleic acid to which they are operably linked, and such vectors are referred to herein as "expression vectors".

The term "host cell" herein refers to a cell into which a foreign nucleic acid is introduced, including the progenies of such a cell. The host cell includes "transformant" and "transformed cell" which include the primary transformed cell and progeny derived therefrom, regardless of the number of passages. The progeny may not be identical to the parental cell in nucleic acid content, and may contain a mutation. The mutant progeny with the same function or biological activity as those screened or selected in the initially transformed cells are included herein.

As used herein, the term "pharmaceutical composition" refers to a preparation that is present in a form which allows the active ingredients contained therein to be biologically effective and does not contain additional ingredients that would be unacceptably toxic to the subject to which the pharmaceutical composition is administered.

The term "treatment" herein refers to surgical or therapeutic treatment, the purpose of which is to prevent and slow down (reduce) an undesired physiological change or pathology, such as cancer progression, in a subject being treated. Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

The term "subject" refers to an organism receiving treatment for a particular disease or disorder as described in the present application. Examples of subjects and patients include mammals, such as humans, primates (e.g., monkeys) or non-primate mammals, receiving treatment for a disease or a disorder.

The term "effective amount" herein refers to an amount of a therapeutic agent effective to prevent or relieve a disease or a disorder or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When the active ingredient is administered to an individual alone, a therapeutically effective dose refers to the ingredient alone. When a certain combination is applied, a therapeutically effective dose refers to the combined dosage of the active ingredients that produce a therapeutic effect, whether administered in combination, sequentially or simultaneously.

The term "cancer" herein refers to or describes the physiological condition in mammals that is typically characterized by unregulated cell growth. Both benign and malignant cancers are included in this definition. The term "tumor" or "neoplasm" herein refers to all neoplastic cell growth and proliferation, whether malignant or benign, and to all pre-cancerous and cancerous cells and tissues. The terms "cancer" and "tumor" are not mutually exclusive when referred to herein.

The term "EC50" herein refers to the half-maximal effective concentration, which includes the concentration of an antibody that induces a response halfway between baseline and maximum after a specified exposure time. EC50 essentially represents the concentration of an antibody at which 50% of the maximal effect thereof is observed and which can be measured by methods known in the art.

### Brief Description of the Figures

FIG. 1 shows the results of the binding of Jurkat cells to antibodies SP34 and OKT3 detected by FACS.
FIG. 2 shows the expression of CD3 on the surface of human T cells detected by antibody SP34.
FIG. 3 shows the expression of CD3 on the surface of monkey T cells detected by antibody SP34.
FIG. 4A-FIG. 4D show the binding of human-murine chimeric antibodies to human T cells detected by FACS.
FIG. 5A-FIG. 5C show the binding of human-murine chimeric antibodies to Jurkat cells detected by FACS.
FIG. 6A-FIG. 6D show the binding of human-murine chimeric antibodies to monkey T cells detected by FACS.
FIG. 7A-FIG. 7D show the activation of signaling pathway of Jurkat cells by human-murine chimeric antibodies detected with luciferase reporter gene.
FIG. 8A-FIG. 8G show the binding activity of humanized antibodies to human CD3ε/δ protein detected by ELISA.
FIG. 9A-FIG. 9G show the binding activity of humanized antibodies to human CD3ε protein detected by ELISA.
FIG. 10A-FIG. 10G show the binding activity of humanized antibodies to human T cells detected by FACS.
FIG. 11A-FIG. 11G show the binding activity of humanized antibodies to monkey CD3ε/δ detected by ELISA.
FIG. 12A-FIG. 12G show the binding activity of humanized antibodies to monkey CD3ε detected by ELISA.
FIG. 13A-FIG. 13G show the binding activity of humanized antibodies to monkey T cells detected by FACS.
FIG. 14A-FIG. 14E show the activity of activation of T cells by humanized antibodies detected by FACS.

### Detailed Description of Embodiments

The present application will be further described below in conjunction with specific examples, and the advantages and characteristics of the present application will become clearer along with the description. If no specific conditions are indicated in the examples, conventional conditions or the conditions suggested by the manufacturer shall be followed. Any reagents or instruments used, unless the manufactures stated, are conventional products that are commercially available.

The examples of the present application are only exemplary and do not limit the scope of the present application in any way. Those skilled in the art should understand that the details and forms of the technical solutions of the present application can be modified or replaced without departing from the spirit and scope of the present application, but these modifications and replacements all fall within the scope of protection of the present application.

### Example 1 Preparation of control antibodies

Positive control antibodies (see Table 1 for details of VH and VL sequences): SP34, OKT3, I2C, 40G5c, F2B and 7221G20 are all antibodies that recognize human CD3, in which SP34, I2C and 40G5c clones recognize a human CD3ε protein, a CD3ε/δ heterodimer and a CD3y/δ heterodimer; and OKT3, F2B and 7221G20 clones only recognize a human CD3ε/δ heterodimer.

The method for preparing the positive control antibody is as follows: the light chain variable region sequences of the positive control antibodies were cloned into the expression vector pcDNA3.4-B1HLK containing a signal peptide and the light chain constant region of human antibody IgG1, respectively, and the heavy chain variable region sequences were cloned into the expression vector pcDNA3.4-B1HH1 containing a signal peptide and the heavy chain constant region of human antibody IgG1, respectively, and thus SP34-hIgG1, OKT3-hIgG1, I2C-hIgG1, 40G5c-hIgG1, 7221G20-hIgG1 and F2B-hIgG1, hereinafter referred to as SP34, OKT3, I2C, 40G5c, 7221G20 and F2B, were obtained. Plasmids were prepared according to the established standard molecular biology methods. For the specific method, see Sambrook, J., Fritsch, E. F. and Maniatis, T. (1989). Molecular Cloning: A Laboratory Manual, Second Edition (Plainview, New York: Cold Spring Harbor Laboratory Press). In addition, the light chain variable region and the heavy chain variable region of the antibody were cloned into the expression vector pcDNA3.4-B1MLK containing a signal peptide and the light chain constant region of murine IgG1, and the expression vector pcDNA3.4-B1MH1 containing a signal peptide and the heavy chain constant region of murine IgG1, respectively, according to the same method. See Table 1 for specific sequence information.

The expression vector was transiently transfected into HEK293E cells (purchased from the Cell Bank of the Committee on Type Culture Collection of Chinese Academy of Sciences) according to the instructions of PEI (purchased from Polysciences, catalog number: 24765-1), the transfected cells were continuously cultured at 37°C for 5 days using FreeStyle TM 293 (Thermofisher scientific, catalog number: 12338018), and the cell components were removed by centrifugation to obtain the culture supernatant containing the antibody. The culture supernatant was loaded onto a protein A chromatography column (the protein A packing AT Protein A Diamond and the chromatography column BXK16/26 were both purchased from Bestchrom (Shanghai) Biosciences Co., Ltd., catalog numbers: AA0273 and B-1620), and the column was washed with a PBS phosphate buffer (pH 7.4), then washed with 20 mM PB and 1 M NaCl (pH 7.2), and finally eluted with a citric acid buffer (pH 3.4). The antibody eluted from the protein A chromatography column was collected, neutralized with 1/10 volume of 1 M Tris (pH 8.0), and dialyzed with PBS at 4°C overnight, and the dialyzed protein was aseptically filtered through a 0.22 micron filter membrane and then subpackaged for storage at -80°C.

The negative control antibody hIgG 1 was the antibody anti-hel-hIgG1 (purchased from Biointron, catalog number: B117901, hereinafter referred to as hIgG 1) against Hen Egg Lysozyme.

The negative control antibody mIgG1 was the antibody anti-hel-mIgG1 (purchased from Biointron, catalog number: B118301, hereinafter referred to as mIgG1) against Hen Egg Lysozyme.

**Table 1 Heavy chain and light chain sequence information of positive antibodies against human CD3**

| **Designation of sequence** | **SEQ ID NO:** | **Amino acid sequence** |
|---|---|---|
| OKT3-VH | 1 | |
| OKT3-VL | 2 | |
| 12C-VH | 3 | EVQLVESGGGLVQPGGSLKLSCAASGFTFNKYAMNWVRQAPGKGL EWVARIRSKYNNYATYYADSVKDRFTISRDDSKNTAYLQMNNLKTE DTAVYYCVRHGNFGNSYISYWAYWGQGTLVTVSS |
| 12C-VL | 4 | QTVVTQEPSLTVSPGGTVTLTCGSSTGAVTSGNYPNWVQQKPGQAP RGLIGGTKFLAPGTPARFSGSLLGGKAALTLSGVQPEDEAEYYCVLW YSNRWVFGGGTKLTVL |
| 40G5c-VH | 5 | EVQLVQSGAEVKKPGASVKVSCKASGYTFTNYYIHWVRQAPGQGL EWIGWIYPGDGNTKYNEKFKGRATLTADTSTSTAYLELSSLRSEDTA VYYCARDSYSNYYFDYWGQGTLVTVSS |
| 40GSc-VL | 6 | DIVMTQSPDSLAVSLGERATINCKSSQSLLNSRTRKNYLAWYQQKPG QPPKLLIYWASTRESGVPDRFSGSGSGTDFTLTISSLQAEDVAVYYCT QSFILRTFGQGTKVEIK |
| 7221G20-VH | 7 | EVQLVESGGGLVQPGRSLRLSCAASGFTFDDYSMHWVRQAPGKGL EWVSGISWNSGSIGYADSVKGRFTISRDNAKNSLYLQMNSLRAEDTA LYYCAKYGSGYGKFYYYGMDVWGQGTTVTVSS |
| 7221G20-VL | 8 | DIQMTQSPSSLSASVGDRVTITCRASQSISSYLNWYQQKPGKAPKLLI YAASSLQSGVPSRFSGSGSGTDFTLTISSLQPEDFATYYCQQSYSTPPI TFGQGTRLEIK |
| F2B-VH | 9 | EVQLVESGGGLVQPGRSLRLSCAASGFTFDDYAMHWVRQAPGKGL EWVSGISWNSGSIGYADSVKGRFTISRDNAKNSLYLQMNSLRAEDTA LYYCAKDSRGYGDYRLGGAYWGQGTLVTVSS |
| F2B-VL | 10 | EIVMTQSPATLSVSPGERATLSCRASQSVSSNLAWYQQKPGQAPRLLI YGASTRATGIPARFSGSGSGTEFTLTISSLQSEDFAVYYCQQYNNWP WTFGQGTKVEIK |
| SP34-VH | 11 | EVQLVESGGGLVQPKGSLKLSCAASGFTFNTYAMNWVRQAPGKGL EWVARIRSKYNNYATYYADSVKDRFTISRDDSQSILYLQMNNLKTED TAMYYCVRHGNFGNSYVSWFAYWGQGTLVTVSS |
| SP34-VL | 12 | QAVVTQESALTTSPGETVTLTCRSSTGAVTTSNYANWVQEKPDHLFT GLIGGTNKRAPGVPARFSGSLIGDKAALTITGAQTEDEAIYFCALWYS NLWVFGGGTKLTVL |
| Human heavy chain constant region | 13 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVD KKVEPKSCDKTHTCPPCPAPELLGGPSVFLFPPKPKDTLMISRTPEVT CVVVDVSHEDPEVKFNWYVDGVEVHNAKTKPREEQYNSTYRVVS VLTVLHQDWLNGKEYKCKVSNKALPAPIEKTISKAKGQPREPQVYT |
| | | LPPSREEMTKNQVSLTCLVKGFYPSDIAVEWESNGQPENNYKTTPPV LDSDGSFFLYSKLTVDKSRWQQGNVFSCSVMHEALHNHYTQKSLSL SPGK |
| Human light chain constant region | 14 | |
| Murine heavy chain constant region | 15 | AKTTPPSVYPLAPGSAAQTNSMVTLGCLVKGYFPEPVTVTWNSGSL SSGVHTFPAVLQSDLYTLSSSVTVPSSTWPSETVTCNVAHPASSTKVD KKIVPRDCGCKPCICTVPEVSSVFIFPPKPKDVLTITLTPKVTCVVVDI SKDDPEVQFSWFVDDVEVHTAQTQPREEQFNSTFRSVSELPIMHQD WLNGKEFKCRVNSAAFPAPIEKTISKTKGRPKAPQVYTIPPPKEQMA KDKVSLTCMITDFFPEDITVEWQWNGQPAENYKNTQPIMDTDGSYF VYSKLNVQKSNWEAGNTFTCSVLHEGLHNHHTEKSLSHSPGK |
| Murine light chain constant region | 16 | RADAAPTVSIFPPSSEQLTSGGASVVCFLNNFYPKDINVKWKIDGSER QNGVLNSWTDQDSKDSTYSMSSTLTLTKDEYERHNSYTCEATHKTS TSPIVKSFNRNEC |

### Example 2 Identification of cells expressing CD3

### 2.1 Identification of cell line expressing CD3 endogenously

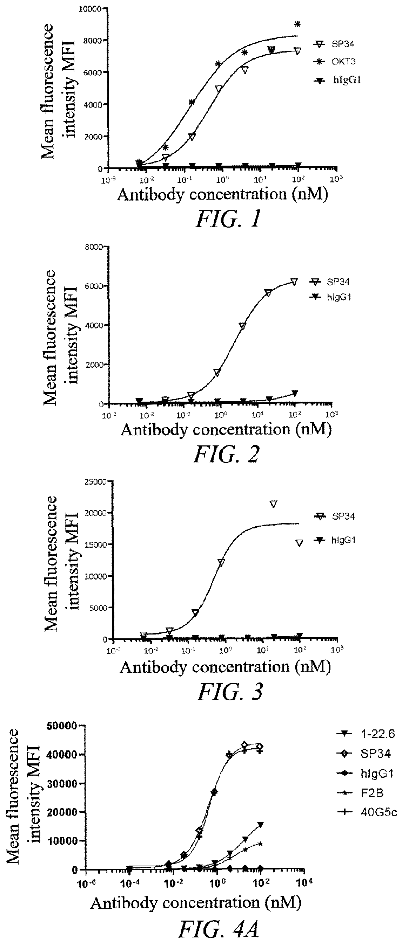

Suspension cells Jurkat cells (purchased from Cell bank of Chinese Academy of Sciences) were scale-up cultured in T-175 cell culture flasks to the logarithmic growth phase, the supernatant of the medium was discarded by centrifugation, and the cell pellet was washed 2 times with PBS. After counting the cells in the previous step, the cell pellet was resuspended with [PBS + 2% (w/v) FBS] blocking solution to 4 × 10⁶ cells/ml, and added to a 96-well FACS reaction plate at 50 µl/well, the supernatant was removed by centrifugation, detection antibodies (initial concentration of 100 nm, gradient dilution at 1 : 5) and an IgG negative control were added at 100 µl/well, the cells were uniformly mixed, and incubated on ice for 1 h. The cells were centrifuged and washed 3 times with a PBS buffer. Alexa647-labeled secondary antibody (purchased from Jackson, catalog number: 109-605-088) was added at 50 µl/well, and the cells were incubated on ice for 1 h. The cells were centrifuged and washed 5 times with a PBS buffer, and FACS (FACS Canto II, purchased from BD Company) was used for detection and result analysis. Data analysis was performed by software (CellQuest) to obtain the mean fluorescence intensity (MFI) of the cells. And then software (GraphPad Prism8) was used for analysis, data fitting, and EC50 value calculation. The detection antibodies were antibodies SP34 and OKT3, and the control was hIgG1. The analysis results are as shown in Table 2 and FIG. 1. The results show that Jurkat cells can bind to SP34 and OKT3, but not to hIgG1, indicating that Jurkat cells can be used as a positive cell for anti-CD3 antibody screening.

**Table 2 Results of endogenous cell line Jurkat cells detected by FACS**

| No. | Designation of antibody | Mean fluorescence intensity of Jurkat cells | |
|---|---|---|---|
| | | Maximum fluorescence value Alexa647 | EC50 (nM) |
| 1 | SP34 | 7327 | 0.45 |
| 2 | OKT3 | 8931 | 0.15 |
| 3 | hIgG1 | 108 | Negative |

### 2.2 Identification of cell line that does not express CD3

MOLT4 cells (purchased from Cell bank of Chinese Academy of Sciences, TCHU224) were scale-up cultured in T-175 cell culture flasks to the logarithmic growth phase, the supernatant of the medium was discarded by centrifugation, and the cell pellet was washed 2 times with PBS. FACS detection and data analysis were performed according to the method in Example 2.1. The detection antibodies were antibodies SP34 and OKT3, and the control was hIgG1. The results are as shown in Table 3. The results show that MOLT4 cells do not bind to SP34 and OKT3, indicating that MOLT4 cells can be used as a negative cell for anti-CD3 antibody screening.

**Table 3 Results of endogenous cell line MOLT4 cells detected by FACS**

| No. | Designation of antibody | Mean fluorescence intensity of MOLT4 cells | |
|---|---|---|---|
| | | Maximum fluorescence value Alexa 647 | EC50 (nM) |
| 1 | SP34 | 211 | Negative |
| 2 | OKT3 | 285 | Negative |
| 3 | hIgG1 | 273 | Negative |

### 2.3 Expansion of human T cells

Human PBMC cells (purchased from AllCells Biotechnology (Shanghai) Co., Ltd.) were subjected to a T cell expansion experiment as described in the instructions of T Cell Activation/Expansion Kit (human, purchased from Miltenyi, catalog number: 130-091-441). When the cells were cultured until 14 days, the supernatant of the medium was discarded by centrifugation, and the cell pellet was washed 2 times with PBS. FACS detection and data analysis were performed using antibody SP34 as primary antibody and Alexa647 labeled secondary antibody (purchased from Jackson Immuno, catalog No. 109-605-098) according to the method of Example 2.1. The analysis results are as shown in Table 4 and FIG. 2. The results show that CD3 positive T cells are obtained after the expansion of human PBMC cells.

**Table 4 Results of human T cells detected by FACS**

| Designation of antibody | Mean fluorescence intensity of T Cells | |
|---|---|---|
| | Maximum fluorescence value Alexa 647 | EC50 (nM) |
| SP34 | 6145 | 2.54 |
| hIgG1 | 480 | Negative |

### 2.4 Expansion of cynomolgus monkey T cells

Cynomolgus monkey PBMC cells (purchased from Shanghai Medicilon Inc., hereinafter referred to as monkey PBMC) were subjected to a cynomolgus monkey (hereinafter referred to as monkey) T cell expansion experiment as described in the instructions of T Cell Activation/Expansion Kit (non-human primate, purchased from Miltenyi, catalog number: 130-092-919). When the cells were cultured until 14 days, the supernatant of the medium was discarded by centrifugation, and the cell pellet was washed 2 times with PBS. FACS detection and data analysis were performed using antibody SP34 as primary antibody and Alexa647 labeled secondary antibody (purchased from Jackson Immuno, catalog No. 109-605-098) according to the method of Example 2.1. The analysis results are as shown in Table 5 and FIG. 3. The results show that CD3 positive T cells are obtained after the expansion of monkey PBMC cells.

**Table 5 Results of monkey T cells detected by FACS**

| Designation of antibody | Mean fluorescence intensity of T Cells | |
|---|---|---|
| | Maximum fluorescence value Alexa 647 | EC50 (nM) |
| SP34 | 21174 | 0.49 |
| hIgG1 | 299 | Negative |

### Example 3 Preparation of anti-human CD3 hybridoma monoclonal antibody

### 3.1 Mouse immunization and hybridoma fusion

Female BALB/c AnNCrl mice, SJL/JorllcoCrl mice, MRL/lpr mice or C57 mice aged 6-8 weeks (all purchased from Shanghai SLAC Laboratory Animal Co., Ltd.) were immunized with a protein containing human CD3ε-ECD (purchased from Sino biological, 10977-H02H) as an immunogen. For the first immunization, the immunogen was emulsified with TiterMax (purchased from Sigma, catalog number: T2684) and injected subcutaneously and intraperitoneally at 0.1 ml respectively, that is, each mouse was injected with 50 micrograms of the immunogen protein. For booster immunization, the immunogen was injected subcutaneously and intraperitoneally at 0.1 ml with Imject Alum adjuvant (purchased from Thermofisher scientific, catalog number: 77161), that is, each mouse was injected with 25 micrograms of the immunogen.

3 days before splenocyte fusion, an antigen solution prepared with normal saline was injected subcutaneously and intraperitoneally at 50 µg/mouse for the final booster immunization. After the splenocytes were isolated, ACK lysing buffer (purchased from Gibco, catalog number: A1049201) was added to lyse the red blood cells doped in the splenocytes to obtain a splenocyte suspension. The cells were centrifuged at 1000 rpm and washed with DMEM basal medium (purchased from Gibco, catalog number: 10569-010) 3 times, and then mixed with mouse myeloma cells SP2/0 (purchased from ATCC, CRL-1581) at a ratio of 2 : 1 in terms of living cells. BTX ECM2001+high-efficiency electrofusion method (see METHODS IN ENZYMOLOGY, VOL. 220) was used for cell fusion. The fused cells were diluted into DMEM medium (purchased from Gibco, catalog number: 10569-010) containing 10% fetal bovine serum (ExCell Bio, catalog number: FSD500) and 1×HAT (purchased from Sigma, catalog number: H0262) (the percentages were volume percentages), then added into a 96-well cell culture plate at 2 × 10⁴/200 microliters per well, and put in a 5% CO₂, 37°C incubator for culture, and the supernatant of the medium after the secretion of the antibody by the hybridoma was used as the supernatant of the hybridoma for positive clone screening.

### 3.2 Screening of mouse hybridoma

The binding of the supernatant of the hybridoma to human CD3ε/δ-His (purchased from Sino Biological, Inc. (China), catalog number: CT038-H2508H) and monkey CD3ε/δ-His (purchased from Acrobiosystems, CDD-C52W4) was detected by ELISA for the screening of the supernatant of the hybridoma (see Example 10.1 below). The binding of the supernatant of the ELISA positive hybridoma clone to Jurkat cells and monkey T cells was detected by flow cytometry, and the activation of Jurkat-Lucia NFAT cells (purchased from InvivoGen, catalog number: jktl-nfat) by the supernatant of the hybridoma was detected by a reporter gene system (see Example 2.1, Example 2.4 and Example 7.1 below for the method). According to the detection results of the supernatant of the hybridoma, the excellent clones (see Table 6-8 for details) were selected, scale-up cultured, and cryopreserved in liquid nitrogen to obtain the hybridoma cells of the present application.

**Table 6 Binding of the supernatant of the hybridoma to human and monkey CD3ε/δ detected by ELISA**

| ELISA(OD₄₅₀) | | |
|---|---|---|
| Clone No. | Human CD3ε/δ | Monkey CD3ε/δ |
| 1-22.6 | 2.17 | 3.08 |
| SP34-mIgG1 | 2.65 | 2.88 |
| mIgG1 | 0.05 | 0.07 |

| Clone No. | Human CD3ε/δ | Monkey CD3ε/δ |
|---|---|---|
| 5-7.13 | 2.08 | 2.09 |
| 5-40.21 | 2.15 | 1.82 |
| SP34-mIgG1 | 1.82 | 1.87 |
| mIgG1 | 0.05 | 0.04 |

| Clone No. | Human CD3ε/δ | Monkey CD3ε/δ |
|---|---|---|
| 6-35.22 | 2.01 | 1.74 |
| 6-44.5 | 2.06 | 1.94 |
| SP34-mIgG1 | 1.79 | 1.63 |
| mIgG1 | 0.04 | 0.05 |

| Clone No. | Human CD3ε/δ | Monkey CD3ε/δ |
|---|---|---|
| 7-35.6 | 1.56 | 1.46 |
| SP34-mIgG1 | 1.40 | 1.57 |
| mIgG1 | 0.04 | 0.04 |

| Clone No. | Human CD3ε/δ | Monkey CD3ε/δ |
|---|---|---|
| 8-18.1 | 2.04 | 2.14 |
| SP34-mIgG1 | 1.79 | 1.84 |
| mIgG1 | 0.04 | 0.04 |

**Table 7 Binding to Jurkat cells and monkey T cells detected by flow cytometry**

| Clone No. | Mean fluorescence intensity of Jurkat cells | Mean fluorescence intensity of monkey T Cells | Mean fluorescence intensity of MOLT4 cells |
|---|---|---|---|
| 1-22.6 | 767 | 20819 | 132 |
| SP34-mIgG1 | 1293 | 22144 | 52 |
| mIgG1 | 92 | 158 | 122 |
| 5-7.13 | 8276 | 16321 | 61 |
| 5-40.21 | 6906 | 15166 | 105 |
| SP34-mIgG1 | 5275 | 7562 | 156 |
| mIgG1 | 152 | 51 | 143 |

| Clone No. | Mean fluorescence intensity of Jurkat cells | Mean fluorescence intensity of monkey T Cells | Mean fluorescence intensity of MOLT4 cells |
|---|---|---|---|
| 6-35.22 | 7048 | 32618 | 124 |
| 6-44.5 | 7240 | 36249 | 98 |
| SP34-mIgG1 | 6388 | 23752 | 124 |
| mIgG1 | 242 | 82 | 107 |

| Clone No. | Mean fluorescence intensity of Jurkat cells | Mean fluorescence intensity of monkey T Cells | Mean fluorescence intensity of MOLT4 cells |
|---|---|---|---|
| 7-35.6 | 284 | 1646 | 44 |
| SP34-mIgG1 | 6034 | 13383 | 180 |
| mIgG1 | 70 | 28 | 80 |

| Clone No. | Mean fluorescence intensity of Jurkat cells | Mean fluorescence intensity of monkey T Cells | Mean fluorescence intensity of MOLT4 cells |
|---|---|---|---|
| 8-18.1 | 895 | 3102 | 69 |
| SP34-mIgG1 | 3141 | 10636 | 75 |
| mIgG1 | 145 | 71 | 56 |

**Table 8 Activation of downstream signaling pathway by anti-CD3 antibodies detected with reporter gene**

| Reporter assay (RLU) | | |
|---|---|---|
| Clone No. | Dilution of the supernatant of the hybridoma by 4 times | Dilution of the supernatant of the hybridoma by 20 times |
| 1-22.6 | 15970 | 8890 |
| SP34-mIgG1 | 5420 | 22840 |
| mIgG1 | 2220 | 2560 |

| Clone No. | Dilution of the supernatant of the hybridoma by 4 times | Dilution of the supernatant of the hybridoma by 20 times |
|---|---|---|
| 5-7.13 | 24510 | 27830 |
| 5-40.21 | 18880 | 15550 |
| SP34-mIgG1 | 15100 | 25250 |
| mIgG1 | 2370 | 1940 |

| Clone No. | Dilution of the supernatant of the hybridoma by 4 times | Dilution of the supernatant of the hybridoma by 20 times |
|---|---|---|
| 6-35.22 | 29050 | 34110 |
| 6-44.5 | 26170 | 21410 |
| SP34-mIgG1 | 38630 | 23640 |
| mIgG1 | 3930 | 5190 |

| Clone No. | Dilution of the supernatant of the hybridoma by 4 times | Dilution of the supernatant of the hybridoma by 20 times |
|---|---|---|
| 7-35.6 | 27450 | 14800 |
| SP34-mIgG1 | 21530 | 38540 |
| mIgG1 | 1760 | 1790 |

| Clone No. | Dilution of the supernatant of the hybridoma by 4 times | Dilution of the supernatant of the hybridoma by 20 times |
|---|---|---|
| 8-18.1 | 26950 | 17770 |
| SP34-mIgG1 | 25370 | 23930 |
| mIgG1 | 1100 | 1200 |

### Example 4 Determination of amino acid sequences of variable regions of light and heavy chains of positive hybridoma clones and production of human-murine chimeric antibody

### 4.1 Determination of amino acid sequences of variable regions of light and heavy chains of positive hybridoma clones

The positive hybridoma cells in the logarithmic growth phase were collected, and were fully lysed with Trizol (Invitrogen, catalog number: 15596-018) and stored at -80°C for testing. For the samples, GENEWIZ Suzhou was entrusted to determine the amino acid sequences of the variable regions of the light and heavy chains of the positive hybridoma clones. The heavy chain variable region and the light chain variable region of the antibodies are as shown in Table 9, and the corresponding CDRs are as shown in Table 10.

**Table 9 Sequences of light chain variable region and heavy chain variable region of antibodies from hybridoma screening**

| SEQ ID NO: | Designation of clone | Sequence |
|---|---|---|
| 17 | 1-22.6-VH | QIPLVQSGPELKKPGETVKISCKTSGYTFTSYGMSWVKQAPGKGLKWMGWI NTYSGVPTYADDFKGRFVFSLDTSASTAFLQINNLKNEDTATYFCARLKNNP FCWGQGSTLTVFS |
| 18 | 1-22.6-VL | DVLMTQSPLSLPVRLGDQASISCRSSQLILHSNGNTYLEWYLQKPGQSPKLLI YKVSNRFSGVPDRFSGSGSGTDFTLKISRVEAEDLGVYYCFQGSHFPWTFGG GTKLEIK |
| 19 | 5-7.13-VH | QVQLQQPGTELVKPGASVKLSCKASGYTFTSYWVHWVKQRPGQGLEWIGN INPSDGVTNYNEKFKSRATLTVDKSSSTAYMQLSSLTSEDSAVYYCTRDAYG QYYFDDWGQGTTLTVSS |
| 20 | 5-7.13-VL | DIVMSQSPSSLVVSAGEKVTMSCKSSQSLLNSRTRKNYLAWYQQKPGQSPK LLIYWASVRDSGVPDRFTGSGSGTDFTLTISNVQAEDLAVYYCIQSYYLRTFG GGTILEIK |
| 21 | 5-40.21-VH | QVQLQQPGSELVRPGASVKLSCKASGYTFTSYWMHWVKQRPGQGLEWIGN TYPGSGSTNYDEKFKSKATLTVDSSSSTAYMQLSSLTSEDSAVYYCTRDRYG VYYFDYWGQGTTLTVSS |
| 22 | 5-40.21-VL | DIVMSQSPSSLAVSAGEKVTMSCKSSQSLLNSRTRKNYLAWYQQKPGQSPKL LIYWASTRESGVPDRFSGSGSGTDFTLTISSVQAEDLAVYYCTQSYNLRTFGG GTKLEIK |
| 23 | 6-35.22-VH | EVKLVESGGGLVQPGASLSLSCAASGFTFTDYYMSWVRQPPGKALEWLAM SRNKAKGHTIEYSSSVKGRFTISRDNSQSILYLQMNALRAEDSATYYCARDT YESYDGYFDVWGAGTTVIVSS |
| 24 | 6-35.22-VL | DIVMSQSPSSLAVSAGETVTMSCKSSQSLFNSRSRKNYLAWYQQKPGQTPKL LIYWASIRESGVPDRFTGSGSGTDFTLTISSVQAEDLAVYYCKQSYYLYTFGS GTKLEIK |
| 25 | 6-44.5-VH | EVQLQQSGPELVKPGASVKMSCKASGHTFTSHIMHWVKQKPGQGLEWIGYI NPNNDRTEYSEKFKGKATLTSDKSSSTAYMELSSLTSEDSAVYYCAGEAYYS NSLYAMDYWGQGTSVTVSS |
| 26 | 6-44.5-VL | DIVMSQSPSSLAVSAGEKVTMSCKSSQSLFNSRTRKNYLAWYQQKPGQSPEL LIYWASTRESGVPVRFTGSGSGTDFTLTISSVQAEDLAVYYCKQSYYLRTFGG GTKLEVK |
| 27 | 7-35.6-VH | EVQLVESGGGLVQPKGSLKLSCAASGFTFNTYAMNWVRQAPGKGLEWVGR IRSKINNYATYYGDSVKDRFTISRDDSQSMFYLQMNNLKTEDTAMYYCVIHE NYGSISWFAYWGQGTLVTVSA |
| 28 | 7-35.6-VL | QAVVTQESTLTTSPGETVTLTCRSSTGAVTTSNYANWVQEKPDHLFTGLIGGT NNRAPGVPARFSGSLIGDKAALTITGAQTEDEAIYFCALWYSNHWVFGGGTK LTVL |
| 29 | 8-18.1-VH | QVQLQQSGAELARPGASVKMSCKASGYIFTMNTMHWVKQRPGQGLEWIG YINPYSGYTKYNQIFKDKATLTADKSSSTAYMRLSSLTSEDSAVYYCLRGGEG VWGQGTTLTVSS |
| 30 | 8-18.1-VL | DVVMTQTPLTLSVTIGQPASISCKSSQSLLDSDGKTYLNWLLQRPGQSPKRLI YLVSELGSGVPDRFTGSGSGTDFTLKISRVEAEDLGVYYCWQGTHFPRTFGG GTKLEIK |

**Table 10 Antibody CDR sequences**

| Designation of antibody | Definition method | CDR1 | CDR2 | CDR3 |
|---|---|---|---|---|
| 1-22.6-VH | Kabat | SYGMS (SEQ ID NO: 31) | WINTYSGVPTYADDFKG (SEQ ID NO: 34) | LKNNPFC (SEQ ID NO: 37) |
| | Chothia | GYTFTSY (SEQ ID NO: 32) | NTYSGV (SEQ ID NO: 35) | LKNNPFC (SEQ ID NO: 37) |
| | IMGT | GYTFTSYG (SEQ ID NO: 33) | INTYSGVP (SEQ ID NO: 36) | ARLKNNPFC (SEQ ID NO: 38) |
| 1-22.6-VL | Kabat | RSSQLILHSNGNTYLE (SEQ ID NO: 39) | KVSNRFS (SEQ ID NO: 41) | FQGSHFPWT (SEQ ID NO: 43) |
| | Chothia | RSSQLILHSNGNTYLE (SEQ ID NO: 39) | KVSNRFS (SEQ ID NO: 41) | FQGSHFPWT (SEQ ID NO: 43) |
| | IMGT | QLILHSNGNTY (SEQ ID NO: 40) | KVS (SEQ ID NO: 42) | FQGSHFPWT (SEQ ID NO: 43) |
| 5-7.13-VH | Kabat | SYWVH (SEQ ID NO: 44) | NINPSDGVTNYNEKFKS (SEQ ID NO: 47) | DAYGQYYFDD (SEQ ID NO: 50) |
| | Chothia | GYTFTSY (SEQ ID NO: 45) | NPSDGV (SEQ ID NO: 48) | DAYGQYYFDD (SEQ ID NO: 50) |
| | IMGT | GYTFTSYW (SEQ ID NO: 46) | INPSDGVT (SEQ ID NO: 49) | TRDAYGQYYFDD (SEQ ID NO: 51) |
| 5-7.13-VL | Kabat | KSSQSLLNSRTRKNYLA (SEQ ID NO: 52) | WASVRDS (SEQ ID NO: 54) | IQSYYLRT (SEQ ID NO: 56) |
| | Chothia | KSSQSLLNSRTRKNYLA (SEQ ID NO: 52) | WASVRDS (SEQ ID NO: 54) | IQSYYLRT (SEQ ID NO: 56) |
| | IMGT | QSLLNSRTRKNY (SEQ ID NO: 53) | WAS (SEQ ID NO: 55) | IQSYYLRT (SEQ ID NO: 56) |
| 5-40.21-VH | Kabat | SYWMH (SEQ ID NO: 57) | NTYPGSGSTNYDEKFKS (SEQ ID NO: 60) | DRYGVYYFDY (SEQ ID NO: 63) |
| | Chothia | GYTFTSY (SEQ ID NO: 58) | YPGSGS (SEQ ID NO: 61) | DRYGVYYFDY (SEQ ID NO: 63) |
| | IMGT | GYTFTSYW (SEQ ID NO: 59) | TYPGSGST (SEQ ID NO: 62) | TRDRYGVYYFDY (SEQ ID NO: 64) |
| 5-40.21-VL | IMGT | KSSQSLLNSRTRKNYLA (SEQ ID NO: 65) | WASTRES (SEQ ID NO: 67) | TQSYNLRT (SEQ ID NO: 69) |
| | Chothia | KSSQSLLNSRTRKNYLA (SEQ ID NO: 65) | WASTRES (SEQ ID NO: 67) | TQSYNLRT (SEQ ID NO: 69) |
| | IMGT | QSLLNSRTRKNY (SEQ ID NO: 66) | WAS (SEQ ID NO: 68) | TQSYNLRT (SEQ ID NO: 69) |
| 6-35.22-VH | Kabat | DYYMS (SEQ ID NO: 70) | MSRNKAKGHTIEYSSSVKG (SEQ ID NO: 73) | DTYESYDGYFDV (SEQ ID NO: 76) |
| | Chothia | GFTFTDY | RNKAKGHT | DTYESYDGYFDV |
| | | (SEQ ID NO: 71) | (SEQ ID NO: 74) | (SEQ ID NO: 76) |
| | IMGT | GFTFTDYY (SEQ ID NO: 72) | SRNKAKGHTI (SEQ ID NO: 75) | ARDTYESYDGYFDV (SEQ ID NO: 77) |
| 6-35.22-VL | Kabat | KSSQSLFNSRSRKNYLA (SEQ ID NO: 78) | WASIRES (SEQ ID NO: 80) | KQSYYLYT (SEQ ID NO: 82) |
| | Chothia | KSSQSLFNSRSRKNYLA (SEQ ID NO: 78) | WASIRES (SEQ ID NO: 80) | KQSYYLYT (SEQ ID NO: 82) |
| | IMGT | QSLFNSRSRKNY (SEQ ID NO: 79) | WAS (SEQ ID NO: 81) | KQSYYLYT (SEQ ID NO: 82) |
| 6-44.5-VH | Kabat | SHIMH (SEQ ID NO: 83) | YINPNNDRTEYSEKFKG (SEQ ID NO: 86) | EAYYSNSLYAMDY (SEQ ID NO: 89) |
| | Chothia | GHTFTSH (SEQ ID NO: 84) | NPNNDR (SEQ ID NO: 87) | EAYYSNSLYAMDY (SEQ ID NO: 89) |
| | IMGT | GHTFTSHI (SEQ ID NO: 85) | INPNNDRT (SEQ ID NO: 88) | AGEAYYSNSLYAMDY (SEQ ID NO: 90) |
| 6-44.5-VL | Kabat | KSSQSLFNSRTRKNYLA (SEQ ID NO: 91) | WASTRES (SEQ ID NO: 93) | KQSYYLRT (SEQ ID NO: 95) |
| | Chothia | KSSQSLFNSRTRKNYLA (SEQ ID NO: 91) | WASTRES (SEQ ID NO: 93) | KQSYYLRT (SEQ ID NO: 95) |
| | IMGT | QSLFNSRTRKNY (SEQ ID NO: 92) | WAS (SEQ ID NO: 94) | KQSYYLRT (SEQ ID NO: 95) |
| 7-35.6-VH | Kabat | TYAMN (SEQ ID NO: 96) | RIRSKINNYATYYGDSVKD (SEQ ID NO: 99) | HENYGSISWFAY (SEQ ID NO: 102) |
| | Chothia | GFTFNTY (SEQ ID NO: 97) | RSKINNYA (SEQ ID NO: 100) | HENYGSISWFAY (SEQ ID NO: 102) |
| | IMGT | GFTFNTYA (SEQ ID NO: 98) | IRSKINNYAT (SEQ ID NO: 101) | VIHENYGSISWFAY (SEQ ID NO: 103) |
| 7-35.6-VL | Kabat | RSSTGAVTTSNYAN (SEQ ID NO: 104) | GTNNRAP (SEQ ID NO: 106) | ALWYSNHWV (SEQ ID NO: 108) |
| | Chothia | RSSTGAVTTSNYAN (SEQ ID NO: 104) | GTNNRAP (SEQ ID NO: 106) | ALWYSNHWV (SEQ ID NO: 108) |
| | IMGT | TGAVTTSNY (SEQ ID NO: 105) | GTN (SEQ ID NO: 107) | ALWYSNHWV (SEQ ID NO: 108) |
| 8-18.1-VH | Kabat | MNTMH (SEQ ID NO: 109) | YINPYSGYTKYNQIFKD (SEQ ID NO: 112) | GGEGV (SEQ ID NO: 115) |
| | Chothia | GYIFTMN (SEQ ID NO: 110) | NPYSGY (SEQ ID NO: 113) | GGEGV (SEQ ID NO: 115) |
| | IMGT | GYIFTMNT (SEQ ID NO: 111) | INPYSGYT (SEQ ID NO: 114) | LRGGEGV (SEQ ID NO: 116) |
| 8-18.1-VL | Kabat | KSSQSLLDSDGKTYLN (SEQ ID NO: 117) | LVSELGS (SEQ ID NO: 119) | WQGTHFPRT (SEQ ID NO: 121) |
| | Chothia | KSSQSLLDSDGKTYLN (SEQ ID NO: 117) | LVSELGS (SEQ ID NO: 119) | WQGTHFPRT (SEQ ID NO: 121) |
| | IMGT | QSLLDSDGKTY (SEQ ID NO: 118) | LVS (SEQ ID NO: 120) | WQGTHFPRT (SEQ ID NO: 121) |

### 4.2 Production of human-murine chimeric antibody

Biointron (Jiangsu) Biological Inc. was entrusted to clone the heavy chain variable region sequences listed in Table 9 into the expression vector pcDNA3.4-B 1HH1 containing a signal peptide and the heavy chain constant region of human antibody IgG1 (the amino acid sequence of the heavy chain constant region is as shown in SEQ ID NO: 13), respectively, and clone the light chain variable region sequences into the expression vector pcDNA3.4-B1HLK containing a signal peptide and the κ light chain constant region of the corresponding human antibody IgG1 (the amino acid sequence of the light chain constant region is as shown in SEQ ID NO: 14) or the expression vector pcDNA3.4-BIHL5 containing a signal peptide and the λ light chain constant region of human antibody IgG1 (the amino acid sequence of the light chain constant region is as shown in SEQ ID NO: 122), respectively, and thus the expression vector of the human-murine chimeric antibody was obtained. The chimeric antibody was expressed and purified according to the method as shown in Example 1.

GQPKANPTVTLFPPSSEELQANKATLVCLISDFYPGAVTVAWKADGSPVK
AGVETTKPSKQSNNKYAASSYLSLTPEQWKSHRSYSCQVTHEGSTVEKTVAPT
ECS (SEQ ID NO: 122).

### Example 5 Identification of anti-CD3 human-murine chimeric antibodies

### 5.1 The binding of chimeric antibodies to different CD3 expressing cells detected by fluorescence-activated cell sorting (FACS)

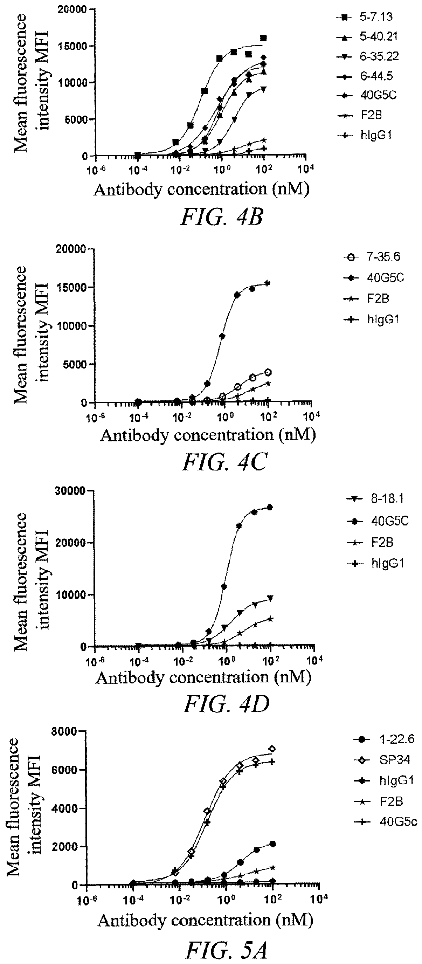

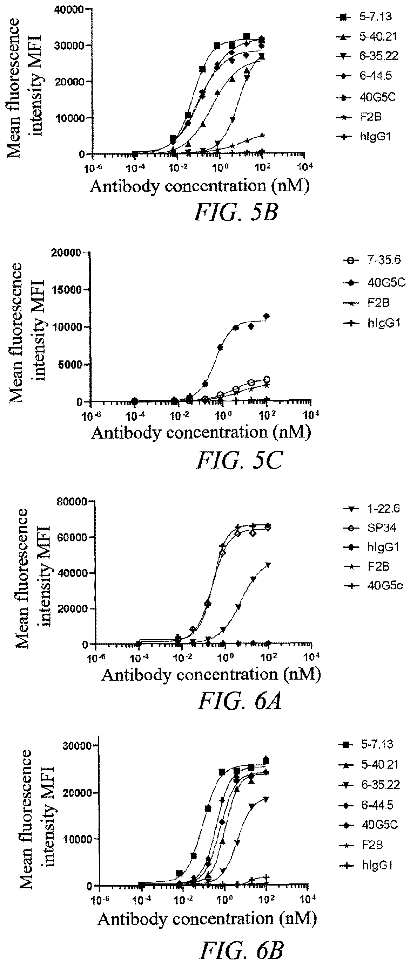

The binding of the chimeric antibodies to CD3 on the cell surface was analyzed by detecting the binding of the antibodies to Jurkat cells and human T cells. Suspension cells Jurkat cells were scale-up cultured in T-75 cell culture flasks to the logarithmic growth phase, the supernatant of the medium was discarded by centrifugation, and the cell pellet was washed 2 times with PBS for later use. The expanded human T cells were washed 2 times with PBS for later use. After counting the cells in the previous step, the cell pellet was resuspended with [PBS + 2% (w/w) BSA] blocking solution to 4 × 10⁶ cells/ml, and added to a 96-well FACS reaction plate at 50 µl/well, and then the chimeric antibody to be tested was added at 50 µl/well, and incubated at 4°C for 1 h. The cells were centrifuged and washed 3 times with a PBS buffer. Alexa Flour 647-labeled secondary antibody (purchased from Jackson Immuno, catalog number: 109-605-098) was added at 50 µl/well, and the cells were incubated on ice for 1 h. The cells were centrifuged and washed 5 times with PBS, and FACS (FACS CantoTM, purchased from BD Company) was used for detection and result analysis. Data analysis was performed by software (CellQuest) to obtain the mean fluorescence density (MFI)of the cells. And then software (GraphPad Prism8) was used for analysis, data fitting, and EC50 value calculation. The same method was used to simultaneously detect the binding of the chimeric antibodies to endogenous CD3 negative cells MOLT4 cells. The analysis results are as shown in Tables 11-1 to 11-4, FIGs. 4A-4D and FIGs. 5A-5C, indicating that the chimeric antibodies can bind to Jurkat cells and human T cells, but not MOLT4 cells, and have good specificity. In addition, compared with the binding of the chimeric antibodies to Jurkat cell line, the binding of the chimeric antibodies to human T cells can better reflect the binding of the antibodies to CD3 on immune cells, so the binding of chimeric antibody 8-18.1 to Jurkat cells was not detected.

**Table 11-1 Binding activity of chimeric antibodies to Jurkat cells, human T cells and MOLT4 cells detected by FACS**

| Designation of antibody | Human T cells | | Jurkat | | MOLT4 |
|---|---|---|---|---|---|
| | EC50 (nM) | MFI_Max | EC50 (nM) | MFI_Max | MFI_Max |
| 1-22.6 | 18.15 | 15126 | 4.28 | 2083 | 124 |
| SP34 | 0.43 | 43060 | 0.13 | 7022 | 570 |
| F2B | 7.56 | 8775 | 8.77 | 841 | 93 |
| 40G5c | 0.46 | 40947 | 0.16 | 6345 | 156 |
| hIgG1 | NB | 148 | NB | 151 | 126 |

| | | | | | |
|---|---|---|---|---|---|
| Notes: NB means not bound. | | | | | |

**Table 11-2 Binding activity of chimeric antibodies to Jurkat cells, human T cells and MOLT4 cells detected by FACS**

| Designation of antibody | Human T cells | | Jurkat | | MOLT4 |
|---|---|---|---|---|---|
| | EC50 (nM) | MFI_Max | EC50 (nM) | MFI_Max | MFI |
| 5-7.13 | 0.10 | 15922 | 0.06 | 32219 | 328 |
| 5-40.21 | 0.99 | 11416 | 0.44 | 26740 | 146 |
| 6-35.22 | 3.62 | 8902 | 8.30 | 26743 | 123 |
| 6-44.5 | 0.62 | 13290 | 0.15 | 31689 | 225 |
| 40G5c | 0.70 | 12335 | 0.10 | 29471 | 168 |
| F2B | 10.44 | 1985 | 20.35 | 4861 | 66 |
| hIgG1 | 19.92 | 823 | NB | 490 | 68 |

| | | | | | |
|---|---|---|---|---|---|
| Notes: NB means not bound. | | | | | |

**Table 11-3 Binding activity of chimeric antibodies to Jurkat cells, human T cells and MOLT4 cells detected by FACS**

| Designation of antibody | Human T cells | | Jurkat | | MOLT4 |
|---|---|---|---|---|---|
| | MFI_Max | EC50 (nM) | MFI_Max | EC50 (nM) | MFI_Max |
| 7-35.6 | 3804 | 4.62 | 2802 | 3.37 | 61 |
| 40G5c | 15415 | 0.66 | 11330 | 0.47 | 59 |
| F2B | 2365 | 15.78 | 2079 | 6.18 | 58 |
| hIgG1 | 195 | NB | 182 | NB | 66 |

| | | | | | |
|---|---|---|---|---|---|
| Notes: NB means not bound. | | | | | |

**Table 11-4 Binding activity of chimeric antibodies to human T cells and MOLT4 cells detected by FACS**

| Designation of antibody | FACS | | |
|---|---|---|---|
| | Human T cells | | MOLT4 |
| | EC50 (nM) | MFI_Max | MFI_Max |
| 8-18.1 | 1.58 | 8982 | 139 |
| 40G5c | 0.99 | 26501 | 161 |
| F2B | 5.71 | 5076 | 81 |
| hIgG1 | NB | 124 | 82 |

| | | | |
|---|---|---|---|
| Notes: NB means not bound. | | | |

### Example 6 Detection of species cross-binding activity of chimeric antibodies

### 6.1 The binding of chimeric antibodies to monkey CD3 expressing cells detected by FACS

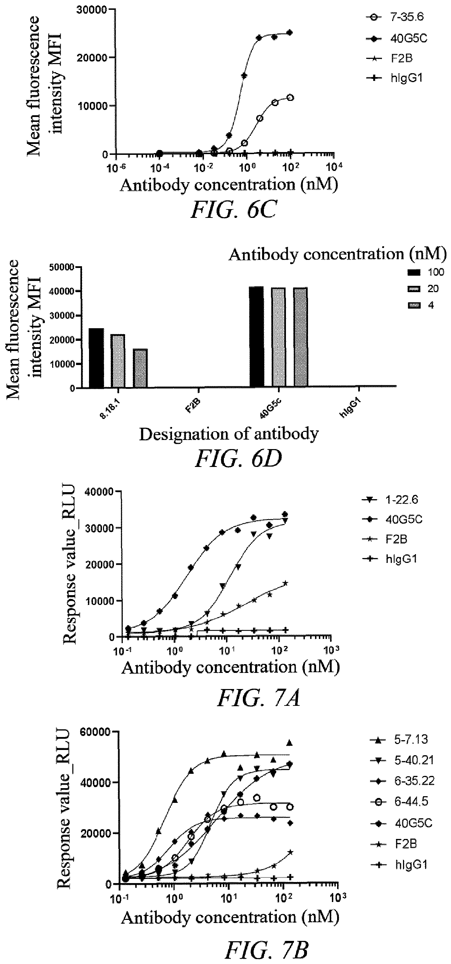

FACS detection and data analysis were performed on the cryopreserved monkey T cells of Example 2.4 according to the method in Example 5.1. The analysis results are as shown in Tables 12-1 to 12-3, and FIGs. 6A-6D, indicating that all chimeric antibodies have the binding activity to monkey T cells.

**Table 12-1 Binding activity of chimeric antibodies to monkey T cells detected by FACS**

| Designation of antibody | Monkey T cells | |
|---|---|---|
| | EC50 (nM) | MFI_Max |
| 1-22.6 | 5.51 | 43563 |
| SP34 | 0.27 | 64682 |
| hIgG1 | NB | 87 |
| F2B | NB | 67 |
| 40G5c | 0.28 | 65994 |

| | | |
|---|---|---|
| Notes: NB means not bound. | | |

**Table 12-2 Binding activity of chimeric antibodies to monkey T cells detected by FACS**

| Designation of antibody | Monkey T cells | |
|---|---|---|
| | EC50 (nM) | MFI_Max |
| 5-7.13 | 0.09 | 26500 |
| 5-40.21 | 1.09 | 24382 |
| 6-35.22 | 4.18 | 18150 |
| 6-44.5 | 0.42 | 27135 |
| 40G5c | 0.63 | 24086 |
| F2B | NB | 296 |
| hIgG1 | NB | 1566 |

| | | |
|---|---|---|
| Notes: NB means not bound. | | |

**Table 12-3 Binding activity of chimeric antibodies to monkey T cells detected by FACS**

| Designation of antibody | Monkey T cells | |
|---|---|---|
| | MFI_Max | EC50 (nM) |
| 7-35.6 | 11512 | 2.74 |
| 40G5c | 24937 | 0.53 |
| F2B | 62 | 25.53 |
| hIgG1 | 195 | NB |

| | | |
|---|---|---|
| Notes: NB means not bound. | | |

**Table 12-4 Binding activity of chimeric antibodies to monkey T cells detected by FACS**

| Designation of antibody | Monkey T cells | | |
|---|---|---|---|
| Antibody concentration | 100 nM | 20 nM | 4 nM |
| 8-18.1 | 24771 | 22143 | 16094 |
| 40G5c | 41398 | 41063 | 40999 |
| F2B | 61 | 60 | 63 |
| hIgG1 | 170 | 80 | 64 |

### Example 7 Functional identification of anti-CD3 chimeric antibody

### 7.1 Function of chimeric antibody detected by luciferase reporter gene (reporter assay)

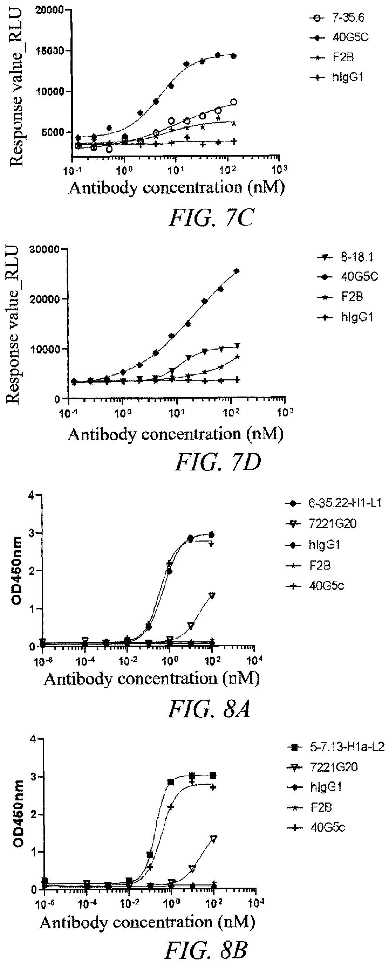

To detect the activation of the signaling pathway in CD3 cells by the chimeric antibodies, the antibody to be detected was diluted with PBS (initial concentration of 133 nM, and gradient dilution at 1 : 2), and then added to a 96-well plate at 100 µl/well. The plate was covered, the cells were incubated at 37°C for 3 h, and then the plate was washed 3 times with PBS. Jurkat-Lucia NFAT (purchased from InvivoGen, catalog number: jktl-nfat) in the logarithmic growth phase were collected, adjusted to the cell concentration of 2e6/mL using a medium (RPMI 1640, purchased from Gibco, catalog number: 12633012) containing 2% FBS, and the cells were added to the washed 96-well plate at 200 µL/well. After incubated at 37°C for 24 h, the cells were centrifuged at 400 g for 5 min, 20 µL of the supernatant was taken into a black opaque 96-well assay plate, 50 µL of a detection reagent (purchased from QUANTI-Luc, brand: Invivogen, catalog number: rep-qlc2) was added, PerkiElmer Ensight microplate reader was used for reading the response value, and then software (GraphPad Prism8) was used for analysis, data fitting, and EC50 value calculation. The results are as shown in Tables 13-1 to 13-4 and FIGs. 7A-7D, all antibodies can activate the downstream signaling pathway of Jurkat-Lucia NFAT cells in the reporter assay.

**Table 13-1 Activation of signaling pathway of Jurkat-Lucia NFAT cells by human-murine chimeric antibodies detected with luciferase reporter gene**

| Designation of antibody | EC50 (nM) | Maximum response value (RLU) |
|---|---|---|
| 1-22.6 | 11.63 | 31670 |
| F2B | 23.7 | 14540 |
| 40G5c | 1.74 | 33440 |
| hIgG1 | Negative | 1830 |

**Table 13-2 Activation of signaling pathway of Jurkat-Lucia NFAT cells by human-murine chimeric antibodies detected with luciferase reporter gene**

| Designation of antibody | EC50 (nM) | Maximum response value (RLU) |
|---|---|---|
| 5-7.13 | 0.70 | 55440 |
| 5-40.21 | 4.79 | 46150 |
| 6-35.22 | 0.82 | 27020 |
| 6-44.5 | 1.84 | 33600 |
| 40G5c | 5.97 | 46910 |
| F2B | The fit is poor | 12040 |
| hIgG1 | Negative | 2330 |

**Table 13-3 Activation of signaling pathway of Jurkat-Lucia NFAT cells by human-murine chimeric antibodies detected with luciferase reporter gene**

| Designation of antibody | EC50 (nM) | Maximum response value (RLU) |
|---|---|---|
| 7-35.6 | 11.81 | 8570 |
| 40G5c | 5.04 | 14320 |
| F2B | 7.41 | 6560 |
| hIgG1 | Negative | 4250 |

**Table 13-4 Activation of signaling pathway of Jurkat-Lucia NFAT cells by human-murine chimeric antibodies detected with luciferase reporter gene**

| Designation of antibody | EC50 (nM) | Maximum response value (RLU) |
|---|---|---|
| 8-18.1 | 11.85 | 10390 |
| 40G5c | 22.49 | 25450 |
| F2B | The fit is poor | 8220 |
| hIgG1 | Negative | 3780 |

### Example 8 Affinity assay of anti-CD3 antibody

### 8.1 Assay of affinity of chimeric antibodies to human CD3ε-His protein

Anti-human CD3 chimeric antibodies were captured using Protein A chip (GE Helthcare; 29-127-558). Sample buffer and running buffer were HBS-EP+ (10 mM HEPES, 150 mM NaCl, 3 mMEDTA, 0.05% surfactant P20) (GE Healthcare; BR-1006-69). The flow-through cell was set to 25°C. The sample block was set to 16°C. Both were pretreated with the running buffer. In each cycle, the antibody to be detected was first captured with a Protein A chip, and then a single concentration of CD3ε antigen protein (purchased from Sino Biological, Inc. (China), catalog number: 10977-H08H) was injected. The binding and dissociation processes of the antibody and the antigen protein were recorded, and finally a glycine solution (pH 1.5, GE Healthcare; BR-1003-54) was used to complete chip regeneration. The binding was measured by injecting different concentrations of recombinant human CD3ε-ECD His proteins in a solution for 240 s with a flow rate of 30 µL/min. The concentration started from 200 nM (see the detailed results for the actual concentration in the test) and was diluted at 1 : 1, making a total of 5 concentrations. The dissociation phase was monitored for up to 600 s and was triggered by switching from sample solution to running buffer. The surface was regenerated by washing with a 10 mM glycine solution (pH 1.5) for 30 s at a flow rate of 30 µL/min. Bulk refractive index difference was corrected by subtracting the response obtained from the goat anti-human Fc surface. Blank injection was also subtracted (= double reference). For calculation of apparent KD and other kinetic parameters, Langmuir 1 : 1 model was used. The binding rate (Ka), dissociation rate (Kd) and binding affinity (KD) of the chimeric antibodies to human CD3ε-His proteins are as shown in Table 14. The affinity of the chimeric antibodies to human CD3 is between 0.1 nM and 10 nM.

**Table 14 Affinity of chimeric antibodies to human CD3ε detected by SPR (biacore)**

| Designation of antibody | Ka (1/Ms) | Kd (1/s) | KD (M) |
|---|---|---|---|
| 1-22.6 | 1.27E+05 | 6.99E-04 | 5.51E-09 |
| 5-40.21 | 6.86E+05 | 3.79E-04 | 5.53E-10 |
| 5-7.13 | 7.85E+05 | 2.70E-04 | 3.45E-10 |
| 6-44.5 | 5.81E+05 | 2.59E-04 | 4.45E-10 |
| 7-35.6 | 2.72E+05 | 2.61E-04 | 9.59E-10 |
| 8-18.1 | 4.45E+05 | 2.41E-04 | 5.42E-10 |
| 40G5c | 1.58E+06 | 3.21E-04 | 2.03E-10 |

### Example 9 Humanized design of antibodies

Humanized design of murine antibodies 6-44.5, 6-35.22, 7-35.6, 8-18.1, 5-7.13, 5-40.21 and 1-22.6: By aligning the IMGT (http://imgt.cines.fr) human antibody heavy and light chain variable region germline gene database, appropriate sequences were selected as humanization light chain templates and humanization heavy chain templates for murine antibodies. First, the CDRs of murine antibodies were grafted into the FRs of the human templates thereof, respectively, to form a variable region sequence in the order of FR1-CDR1-FR2-CDR2-FR3-CDR3-FR4. According to the three-dimensional structure simulation of the antibody, the key amino acids in the FR region sequence of the humanized antibody were back-mutated to the corresponding amino acids of the murine antibody to ensure the original affinity. In addition, according to particular needs, some amino acids in the CDR region sequences of the humanized antibody were mutated to the corresponding homologous amino acids of the human template to improve the degree of humanization, or to other amino acids to optimize the physicochemical properties of the molecule (for example, hotspot mutation is performed on a site that is prone to deamidation modification, glycosylation modification and isomerization), thereby obtaining the humanized antibody. The CDR regions of the antibody in this example were determined and annotated by the Kabat numbering system (see http://www.abysis.org/abysis/sequence_input/key_annotation/key_annotation.cgi). Among them, See Table 15 for the corresponding humanization template for the murine antibody, Table 16 for the corresponding VL and VH for the final humanized antibody, Table 17 for the specific sequences, Table 18 for a part of the CDRs (with hotspot mutation) of the humanized antibody sequences, and the remaining CDR sequences are unchanged before and after humanization, and are not re-listed here. The humanized antibodies were produced according to Example 4.2.

**Table 15 Corresponding humanization templates for murine antibodies**

| Murine antibody clone no. | Humanization light chain template | Humanization heavy chain template |
|---|---|---|
| 6-44.5 | IGKV2-40*01 + IGKJ4*01 | IGHV1-69*02 + IGHJ6*01 |
| 6-35.22 | IGKV4-1*01 + IGKJ2*01 | IGHV3-30*01 + IGHJ6*01 |
| 7-35.6 | IGLV7-43*01 + IGLJ2*01 | IGHV3-73*01 + IGHJ1*01 |
| 8-18.1 | IGKV2-30*01 + IGKJ4*01 | IGHV1-3*01 + IGHJ6*01 |
| 5-7.13 | IGKV4-1*01, IGKV2-40*01 and IGKJ4*01 | IGHV1-3*01 and IGHJ6*01 |
| 5-40.21 | IGKV4-1*01, IGKV2-40*01 and IGKJ4*01 | IGHV1-3*01 and IGHJ6*01 |
| 1-22.6 | IGKV2-40*01, IGKV4-1*01 and IGKJ4*01 | IGHV7-4-1*02 and IGHJ6*01 |

**Table 16 Corresponding VLs and VHs of humanized antibodies**

| Humanized antibody | VL | VH |
|---|---|---|
| 6-44.5-H1-L2 | 6-44.5-L2 (SEQ ID NO: 123) | 6-44.5-H1 (SEQ ID NO: 124) |
| 6-35.22-H1-L1 | 6-35.22-L1 (SEQ ID NO: 125) | 6-35.22-H1 (SEQ ID NO: 126) |
| 7-35.6-H2-L3 | 7-35.6-L3 (SEQ ID NO: 127) | 7-35.6-H2 (SEQ ID NO: 128) |
| 8-18.1-H1-L2 | 8-18.1-L2 (SEQ ID NO: 129) | 8-18.1-H1 (SEQ ID NO: 130) |
| 5-7.13-H1a-L2 | 5-7.13-L2 (SEQ ID NO: 131) | 5-7.13-H1a (SEQ ID NO: 132) |
| 5-40.21-H3-L1 | 5-40.21-L1 (SEQ ID NO: 133) | 5-40.21-H3 (SEQ ID NO: 134) |
| 1-22.6-1-H2a-L2a | 1-22.6-1-L2a (SEQ ID NO: 135) | 1-22.6-1-H2a (SEQ ID NO: 136) |

**Table 17 VL and VH sequences of humanized antibodies**

| No. | Sequence |
|---|---|
| 6-44.5-L2 | DIVMTQSPDSLAVSLGERATINCKSSQSLFNSRTRKNYLAWYQQKPGQSPKLLIYWAS TRESGVPDRFSGSGSGTDFTLTISSLQAEDVAVYYCKQSYYLRTFGGGTKVEIK(SEQ ID NO: 123) |
| 6-44.5-H1 | EVQLVQSGAEVKKPGSSVKVSCKASGHTFSSHIMHWVRQAPGQGLEWMGYINPNND RTEYSEKFKGRVTITADKSTSTAYMELSSLRSEDTAVYYCAGEAYYSNSLYAMDYWGQ GTTVTVSS(SEQ ID NO: 124) |
| 6-35.22-L1 | DIVMTQSPDSLAVSLGERATINCKSSQSLFNSRSRKNYLAWYQQKPGQTPKLLIYWASI RESGVPDRFSGSGSGTDFTLTISSLQAEDVAVYYCKQSYYLYTFGQGTKLEIK(SEQ ID NO: 125) |
| 6-35.22-H1 | EVQLVESGGGVVQPGRSLRLSCAASGFTFTDYYMSWVRQAPGKGLEWVAMSRNKA KGHTIEYSSSVKGRFTISRDNSKNTLYLQMNSLRAEDTAVYYCARDTYESYDGYFDVW GQGTTVTVSS(SEQ ID NO: 126) |
| 7-35.6-L3 | ETVVTQEPSLTVSPGGTVTLTCRSSTGAVTTSNYANWVQQKPGQAFTGLIGGTNNRAP WTPARFSGSLLGGKAALTLSGVQPEDEAEYYCALWYSNHWVFGGGTKLTVL(SEQ ID NO: 127) |
| 7-35.6-H2 | EVQLVESGGGLVQPGGSLKLSCAASGFTFNTYAMNWVRQASGKGLEWVGRIRSKINN YATYYGDSVKDRFTISRDDSKNTFYLQMNSLKTEDTAVYYCVIHENYGSISWFAYWGQ GTLVTVSS(SEQ ID NO: 128) |
| 8-18.1-L2 | DVVMTQSPLSLPVTLGQPASISCKSSQSLLDSDGKTYLNWLQQRPGQSPRRLIYLVSEL GSGVPDRFSGSGSGTDFTLKISRVEAEDVGVYYCWQGTHFPRTFGGGTKVEIK(SEQ ID NO: 129) |
| 8-18.1-H1 | EVQLVQSGAEVKKPGASVKVSCKASGYTFTMNTMHWVRQAPGQRLEWMGYINPYS GYTKYNQIFKDRVTITADKSASTAYMELSSLRSEDTAVYYCLRGGEGVWGQGTTVTVS S(SEQ ID NO: 130) |
| 5-7.13-L2 | DIVMTQTPLSLPVTPGEPASISCKSSQSLLNSRTRKNYLAWYLQKPGQSPQLLIYWASV RDSGVPDRFSGSGSGTDFTLKISRVEAEDVGVYYCIQSYYLRTFGGGTKVEIK(SEQ ID NO: 131) |
| 5-7.13-H1a | EVQLVQSGAEVKKPGASVKVSCKASGYTFTSYWVHWVRQAPGQRLEWMGNINPSD GVT NYNEKFKSRVTITVDKSASTAYMELSSLRSEDTAVYYCTRDAYGQYYFDNWGQGTTVT VSS (SEQ ID NO: 132) |
| 5-40.21-L1 | DIVMTQSPDSLAVSLGERATINCKSSQSLLNSRTRKNYLAWYQQKPGQSPKLLIYWAS TRESGVPDRFSGSGSGTDFTLTISSLQAEDVAVYYCTQSYNLRTFGGGTKVEIK(SEQ ID NO: 133) |
| 5-40.21-H3 | EVQLVQSGAEVKKPGASVKVSCKASGYTFTSYWMHWVRQAPGQGLEWMGNTYPGS GSTNYDEKFKSRVTITVDSSASTAYMELSSLRSEDTAVYYCTRDRYGVYYFDYWGQGT TVTVSS (SEQ ID NO: 134) |
| 1-22.6-1-L2a | DIVMTQSPDSLAVSLGERATINCRSSQLILHSSGNTYLEWYQQKPGQSPKLLIYKVSNR FSGVPDRFSGSGSGTDFTLTISSLQAEDVAVYYCFQGSHFPWTFGGGTKVEIK(SEQ ID NO: 135) |
| 1-22.6-1-H2a | EVQLVQSGSELKKPGASVKVSCKTSGYTFTSYGMSWVRQAPGQGLEWMGWINTYSG VPTYAQDFKGRFVFSLDTSVSTAYLQISSLKAEDTAVYYCARLKNNPFVWGQGTTVT VSS(SEQ ID NO: 136) |

**Table 18 A part of CDR sequences of humanized antibodies**

| Designation of antibody | Definiti on method | CDR1 | CDR2 | CDR3 |
|---|---|---|---|---|
| 1-22.6-1-H2a (hotspot repair) | Kabat | SYGMS (SEQ ID NO: 31) | WINTYSGVPTYA**Q**DFKG (SEQ ID NO: 137) | LKNNPF**V** (SEQ ID NO: 138) |
| | Chothia | GYTFTSY | NTYSGV | LKNNPF**V** |
| | | (SEQ ID NO: 32) | (SEQ ID NO: 35) | (SEQ ID NO: 138) |
| | IMGT | GYTFTSYG (SEQ ID NO: 33) | INTYSGVP (SEQ ID NO: 36) | ARLKNNPF**V** (SEQ ID NO: 139) |
| 1-22.6-1-L2a (hotspot repair) | Kabat | RSSQLILHS**S**GNTYLE (SEQ ID NO: 140) | KVSNRFS (SEQ ID NO: 41) | FQGSHFPWT (SEQ ID NO: 43) |
| | Chothia | RSSQLILHS**S**GNTYLE (SEQ ID NO: 140) | KVSNRFS (SEQ ID NO: 41) | FQGSHFPWT (SEQ ID NO: 43) |
| | IMGT | QLILHS**S**GNTY (SEQ ID NO: 141) | KVS (SEQ ID NO: 42) | FQGSHFPWT (SEQ ID NO: 43) |
| 5-7.13-H1a (hotspot repair) | Kabat | SYWVH (SEQ ID NO: 44) | NINPSDGVTNYNEKFKS (SEQ ID NO: 47) | DAYGQYYFD**N** (SEQ ID NO: 142) |
| | Chothia | GYTFTSY (SEQ ID NO: 45) | NPSDGV (SEQ ID NO: 48) | DAYGQYYFD**N** (SEQ ID NO: 142) |
| | IMGT | GYTFTSYW (SEQ ID NO: 46) | INPSDGVT (SEQ ID NO: 49) | TRDAYGQYYFD**N** (SEQ ID NO: 143) |

| | | | | |
|---|---|---|---|---|
| Notes: Boxed character is a mutation site for hotspot repair. | | | | |

### Example 10 Identification of binding ability of humanized antibodies to human CD3ε

### 10.1 The binding of antibodies to CD3 protein detected by enzyme-linked immunosorbent assay (ELISA)

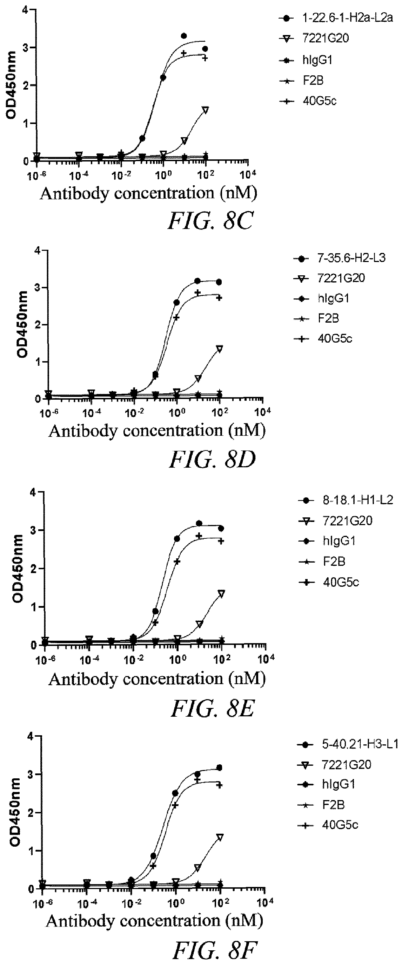

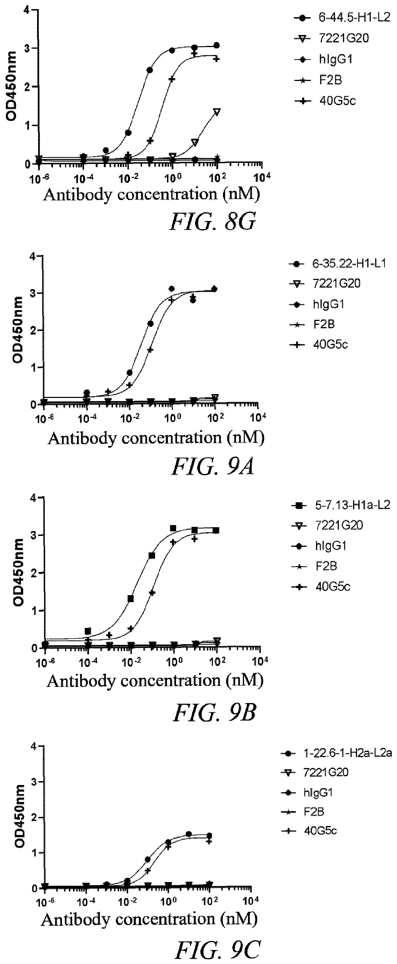

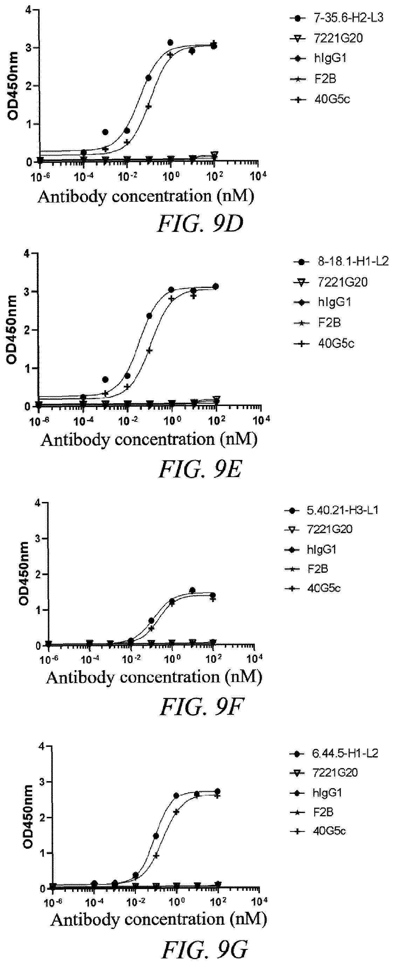

To detect the binding activity of the humanized antibodies to CD3 protein, CD3 protein was diluted with PBS to the final concentration of 1 µg/mL, then added to a 96-well ELISA plate at 50 µL/well, and the plate was sealed with a plastic film, and incubated overnight at 4°C. On the next day, the plate was washed 2 times with PBS, and a blocking solution [PBS + 2% (v/v) BSA] was added for blocking at room temperature for 2 h. The blocking solution was poured off, and the antibody (initial concentration of 100 nM, and gradient dilution at 1 : 10) or a negative control antibody was added at 50 µl/well. After incubation at 37°C for 2 h, the plate was washed 3 times with PBS. HRP (horseradish peroxidase)-labeled secondary antibody (purchased from Jackson, catalog number: 309-035-088) was added, and incubated at 37°C for 0.5 h, and the plate was washed 5 times with PBS. TMB substrate was added at 50 µl/well, and incubated at room temperature for 10 min, and then a stop solution (1.0 N HCl) was added at 50 µl/well. The OD450 nm values were read by an ELISA microplate reader (Multimode Plate Reader, EnSight, purchased from Perkin Elmer). The experimental results are as shown in FIGs. 8A-8G, FIGs. 9A-9G and Table 19. The IgG control was hIgG1, and the positive controls were 40G5c, 7221G20 and F2B. The data in the table are OD450 nm values. The results show that all humanized antibodies have the binding activity to CD3ε protein or CD3ε/δ protein in the ELISA experiments.

**Table 19-1 Binding activity of humanized antibodies to human CD3ε/δ and CD3ε proteins at ELISA level**

| Designation of antibody | Human CD3ε/δ | | Human CD3ε | |
|---|---|---|---|---|
| | max_OD450 | EC50 (nM) | max_OD450 | EC50 (nM) |
| 6-35.22-H1-L1 | 2.93 | 0.53 | 3.10 | 3.37E-02 |
| 7-35.6-H2-L3 | 3.15 | 0.32 | 3.12 | 3.95E-02 |
| 8-18.1-H1-L2 | 3.16 | 0.22 | 3.11 | 3.63E-02 |
| 5-7.13-H1a-L2 | 3.01 | 0.19 | 3.16 | 2.06E-02 |
| 40G5c | 2.83 | 0.36 | 3.09 | 1.18E-01 |
| F2B | 0.16 | Negative | 0.10 | Negative |
| 7221G20 | 1.31 | 23.45 | 0.15 | Negative |
| hIgG1 | 0.09 | Negative | 0.11 | Negative |

**Table 19-2 Binding activity of humanized antibodies to human CD3ε/δ and CD3ε proteins at ELISA level**

| Designation of antibody | Human CD3ε/δ | | Human CD3ε | |
|---|---|---|---|---|
| | max_OD450 | EC50 (nM) | max_OD450 | EC50 (nM) |
| 5-40.21-H3-L1 | 3.15 | 0.28 | 1.53 | 0.13 |
| 1-22.6-H2a-L2a | 3.29 | 0.45 | 1.49 | 0.10 |
| 40G5c | 2.83 | 0.36 | 1.49 | 0.22 |
| F2B | 0.16 | Negative | 0.05 | Negative |
| 7221G20 | 1.31 | 23.45 | 0.05 | Negative |
| hIgG1 | 0.09 | Negative | 0.09 | Negative |

**Table 19-3 Binding activity of humanized antibodies to human CD3ε/δ and CD3ε proteins at ELISA level**

| Designation of antibody | Human CD3ε/δ | | Human CD3ε | |
|---|---|---|---|---|
| | max_OD450 | EC50 (nM) | max_OD450 | EC50 (nM) |
| 6-44.5-H1-L2 | 3.05 | 0.03 | 2.70 | 0.09 |
| 40G5c | 2.83 | 0.36 | 2.59 | 0.22 |
| F2B | 0.16 | Negative | 0.16 | Negative |
| 7221G20 | 1.31 | 23.45 | 0.06 | Negative |
| hIgG1 | 0.09 | Negative | 0.09 | Negative |

### 10.2 The binding of antibodies to CD3 expressing cells detected by fluorescence-activated cell sorting (FACS)

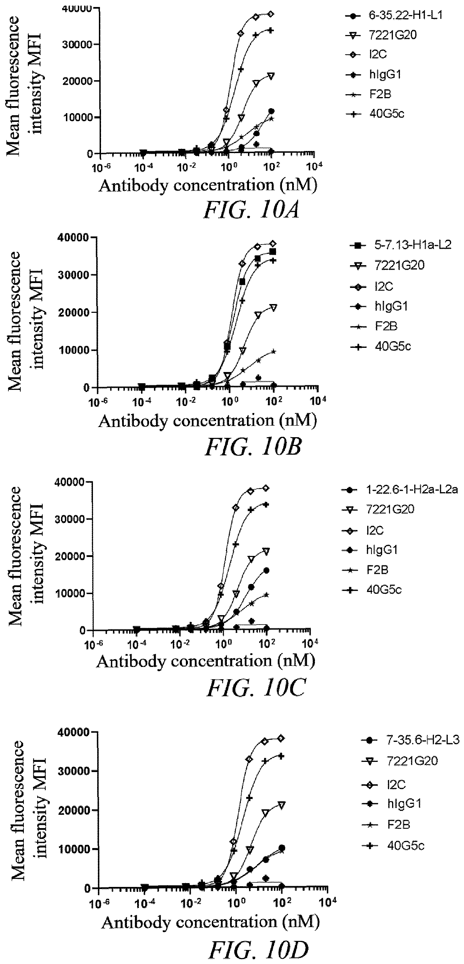

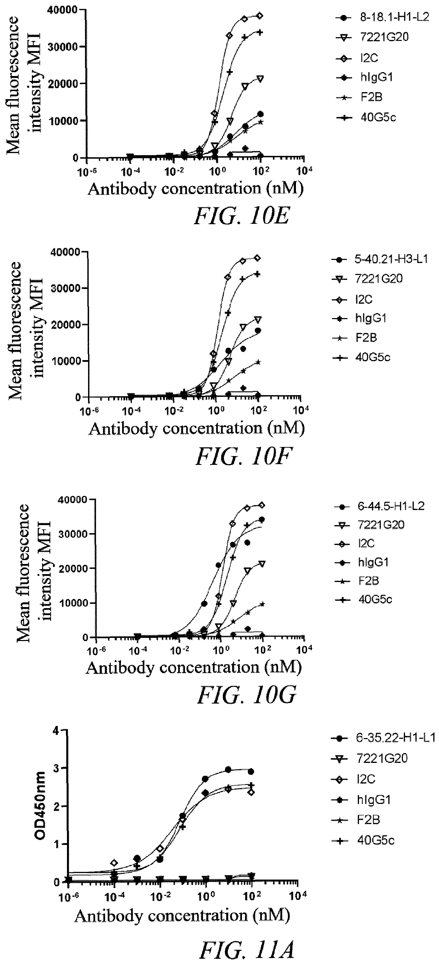

The expanded human PBMC cells were collected, and FACS detection and data analysis were performed according to the method in Example 5.1. The analysis results are as shown in FIGs. 10A-10G and Table 20, wherein the IgG control was hIgG1, and the positive controls were 40G5c, I2C, 7221G20 and F2B. The results show that the humanized antibodies specifically bind to CD3 on the surface of human T cells.

**Table 20 Binding activity of humanized antibodies to CD3 expressing positive and negative cells at FACS level**

| Designation of antibody | Human T cells | | MOLT4 |
|---|---|---|---|
| | max_MFI | EC50 (nM) | max_MFI |
| 6-35.22-H1-L1 | 11349 | 49.66 | 80 |
| 1-22.6-1-H2a-L2a | 15865 | 11.04 | 77 |
| 7-35.6-H2-L3 | 10003 | 9.45 | 80 |
| 8-18.1-H1-L2 | 11482 | 7.05 | 124 |
| 5-40.21-H3-L1 | 18044 | 1.65 | 81 |
| 5-7.13-H1a-L2 | 35913 | 1.52 | 149 |
| 6-44.5-H1-L2 | 33953 | 0.43 | 96 |
| 40G5c | 33491 | 2.09 | 110 |
| I2C | 38011 | 1.32 | 244 |
| 7221G20 | 20875 | 4.72 | 72 |
| F2B | 9273 | 7.03 | 70 |
| hIgG1 | 2249 | Negative | 76 |

### 10.3 Affinity of antibodies to human CD3ε protein detected by surface plasmon resonance (SPR)

The affinity of the humanized antibodies to CD3ε protein was determined by surface plasmon resonance (SPR), with the experimental method as follows: anti-human CD3 antibodies were captured using Protein A chip (GE Helthcare; 29-127-558). Running buffer was HBS-EP+ (10 mM HEPES, 150 mM NaCl, 3 mM EDTA, 0.05% surfactant P20) (GE Healthcare; BR-1006-69). The flow-through cell was set to 25°C. The sample block was set to 16°C. Both were pretreated with the running buffer. In each cycle, an antibody to be detected was first captured with a Protein A chip, and then a single concentration of CD3ε antigen protein was injected. The binding and dissociation processes of the antibody and the antigen protein were recorded, and finally Glycine pH 1.5 (GE Healthcare; BR-1003-54) was used to complete chip regeneration. The binding was measured by injecting different concentrations of recombinant human CD3ε proteins in a solution for 240 s with a flow rate of 30 µL/min. The concentration started from 200 nM (see the detailed results for the actual concentration in the test) and was diluted at 1 : 1, making a total of 5 concentrations. The dissociation phase was monitored for up to 600 s and was triggered by switching from sample solution to running buffer. The surface was regenerated by washing with a 10 mM glycine solution (pH 1.5) for 30 s at a flow rate of 30 µL/min. Bulk refractive index difference was corrected by subtracting the response obtained from the goat anti-human Fc surface, and the blank injection was subtracted at the same time (= double reference). For calculation of apparent KD value and other kinetic parameters, Langmuir 1:1 model was used. The binding rate (Ka), dissociation rate (Kd) and binding affinity (KD) of the humanized antibodies to human CD3ε protein are as shown in Tables 21-22. The affinity of all humanized antibodies detected to human CD3ε is about 1 nM, and the affinity of all humanized antibodies detected to human CD3ε/δ is between 10 nM and 200 nM.

**Table 21 Affinity of humanized antibodies to human CD3ε protein detected by SPR**

| Designation of antibody | Ka (1/Ms) | Kd (1/s) | KD (M) |
|---|---|---|---|
| 6-44.5-H1-L2 | 4.66E+05 | 3.11E-04 | 6.69E-10 |
| 7-35.6-H2-L3 | 2.86E+05 | 3.69E-04 | 1.29E-09 |
| 1-22.6-1-H2a-L2a | 8.36E+04 | 3.05E-04 | 3.64E-09 |
| 5-7.13-H1a-L2 | 2.66E+05 | 2.14E-04 | 8.06E-10 |
| 5-40.21-H3-L1 | 2.60E+05 | 4.85E-04 | 1.87E-09 |
| 8-18.1-H1-L2 | 2.74E+05 | 2.49E-04 | 9.08E-10 |

**Table 22 Affinity of humanized antibodies to human CD3ε/δ protein detected by SPR**

| Designation of antibody | Ka (1/Ms) | Kd (1/s) | KD (M) |
|---|---|---|---|
| 1-22.6-1-H2a-L2a | 1.19E+05 | 1.34E-02 | 1.13E-07 |
| 5-7.13-H1a-L2 | 3.58E+05 | 9.83E-03 | 2.74E-08 |
| 5-40.21-H3-L1 | 1.61E+05 | 1.84E-02 | 1.15E-07 |
| 6-44.5-H1-L2 | 4.91E+05 | 3.39E-02 | 6.90E-08 |
| 8-18.1-H1-L2 | 4.69E+05 | 3.17E-02 | 6.74E-08 |
| 7-35.6-H2-L3 | 2.31E+05 | 3.35E-02 | 1.45E-07 |
| 40G5c | 1.53E+06 | 3.27E-02 | 2.13E-08 |
| 12C | 2.50E+05 | 1.35E-03 | 5.39E-09 |

### Example 11 Identification of cross-binding activity of humanized antibodies

### 11.1 The binding of antibodies to monkey CD3 protein detected by enzyme-linked immunosorbent assay (ELISA)

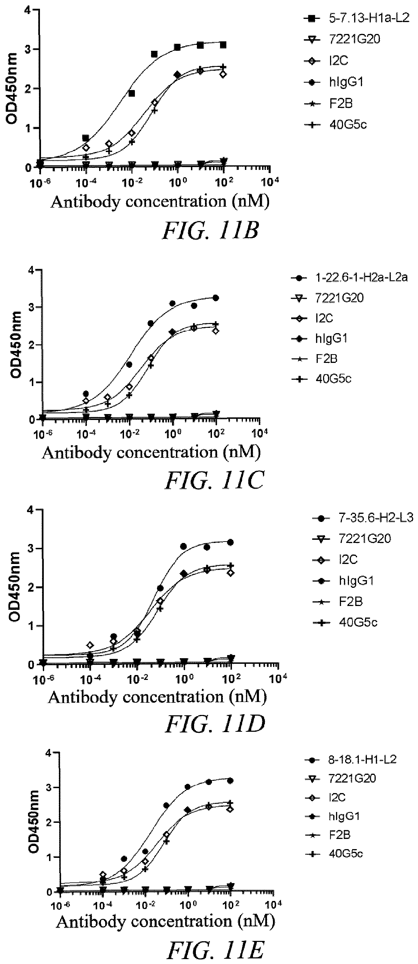

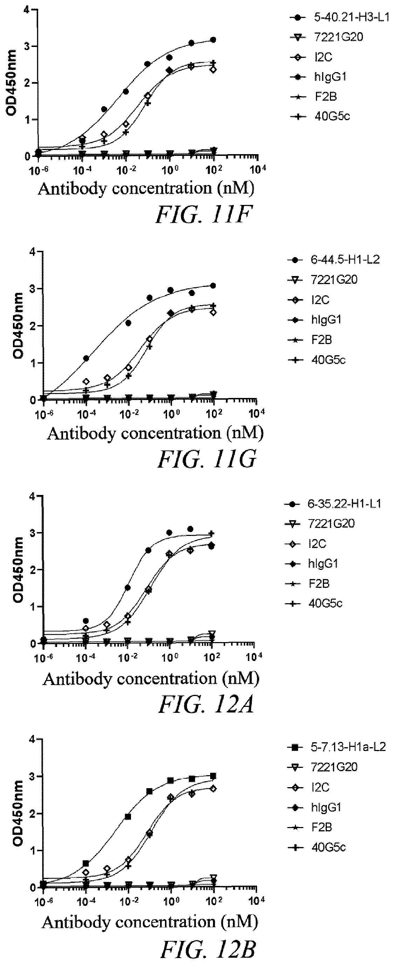

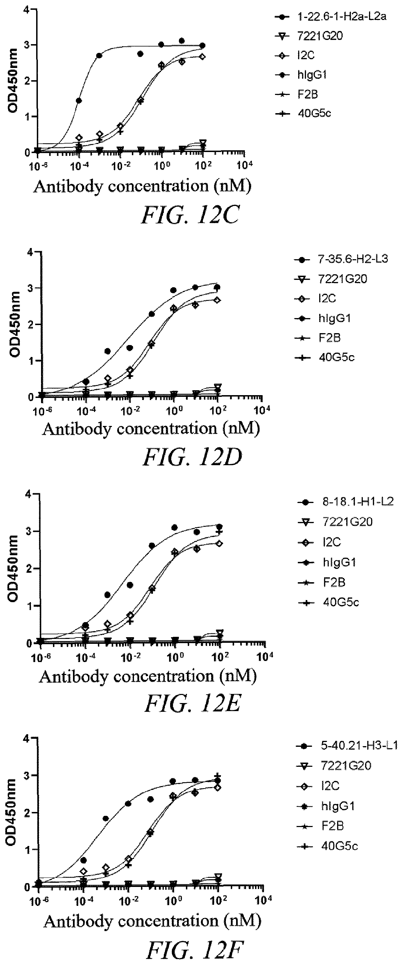

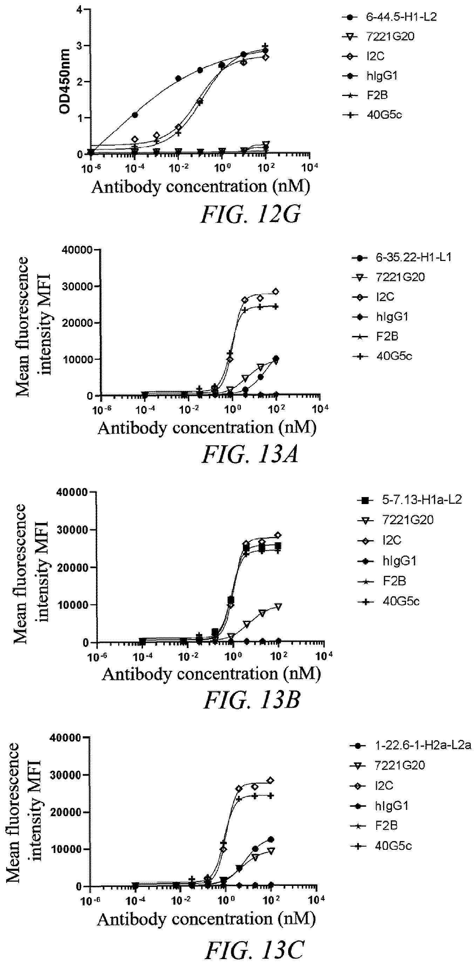

To detect the binding activity of the humanized antibodies to monkey CD3 protein, CD3 protein was diluted with PBS to the final concentration of 1 µg/mL, and then added to a 96-well ELISA plate at 50 µl/well. The binding activity of the humanized antibodies to monkey CD3 protein was detected according to the ELISA assay method in Example 10.1, in which the IgG control was hIgG1, and the positive controls were 40G5c, I2C, 7221G20 and F2Bs. The analysis results are as shown in FIGs. 11A-11G, FIGs. 12A-12G and Table 23. The data in the table are OD450 nm values. The results show that all humanized antibodies have the binding activity to monkey CD3 protein in the ELISA experiments.

**Table 23 Binding activity of humanized antibodies to monkey CD3ε/δ and CD3ε proteins at ELISA level**

| Designation of antibody | Monkey CD3ε/δ | | Monkey CD3ε | |
|---|---|---|---|---|
| | max_OD450 | EC50 (nM) | max_OD450 | EC50 (nM) |
| 6-35.22-H1-L1 | 2.94 | 7.82E-02 | 3.08 | 1.18E-02 |
| 1-22.6-1-H2a-L2a | 3.23 | 1.24E-02 | 3.09 | 1.06E-04 |
| 7-35.6-H2-L3 | 3.13 | 5.52E-02 | 3.01 | 8.59E-03 |
| 8-18.1-H1-L2 | 3.16 | 1.93E-02 | 3.10 | 4.75E-03 |
| 5-40.21-H3-L1 | 3.16 | 4.56E-03 | 2.84 | 4.67E-04 |
| 5-7.13-H1a-L2 | 3.08 | 3.32E-03 | 2.99 | 2.63E-03 |
| 6-44.5-H1-L2 | 3.07 | 2.25E-04 | 2.84 | 9.47E-06 |
| 40G5c | 2.52 | 7.54E-02 | 2.96 | 1.28E-01 |
| I2C | 2.42 | 3.60E-02 | 2.65 | 7.87E-02 |
| F2B | 0.05 | Negative | 0.06 | Negative |
| 7221G20 | 0.11 | Negative | 0.23 | Negative |
| hIgG1 | 0.15 | Negative | 0.16 | Negative |

### 11.2 The binding of antibodies to monkey T cells detected by fluorescence-activated cell sorting (FACS)

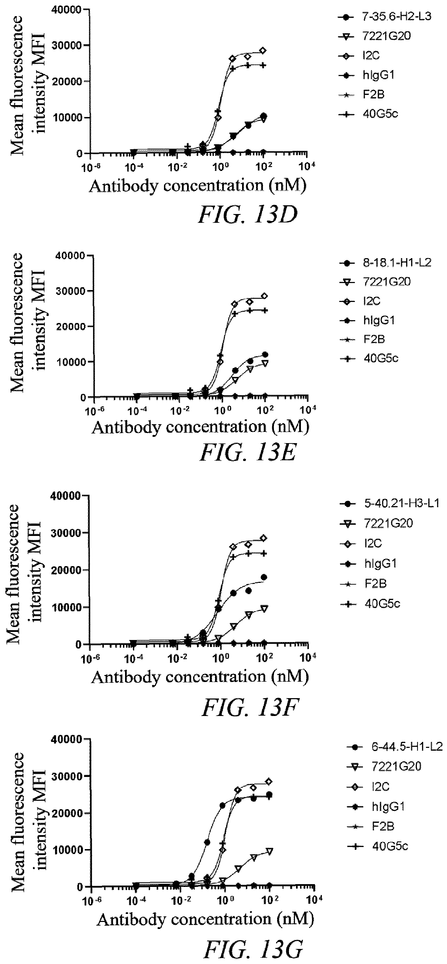

The expanded monkey T cells were collected, and FACS detection and data analysis were performed according to the method in Example 5.1. The analysis results are as shown in FIGs. 13A-13G and Table 24, wherein the IgG control was hIgG1, and the positive controls were 40G5c, I2C, 7221G20 and F2B. The results show that all humanized antibodies have the specific binding activity to CD3 on the surface of monkey T cells.

**Table 24 Binding of humanized antibodies to monkey T cells**

| Designation of antibody | Monkey T cells | |
|---|---|---|
| | max_MFI | EC50 (nM) |
| 6-35.22-H1-L1 | 9957 | 35.42 |
| 1-22.6-1-H2a-L2a | 12287 | 6.92 |
| 7-35.6-H2-L3 | 10162 | 7.84 |
| 8-18.1-H1-L2 | 11751 | 2.84 |
| 5-40.21-H3-L1 | 17764 | 0.73 |
| 5-7.13-H1a-L2 | 25448 | 0.92 |
| 6-44.5-H1-L2 | 24775 | 0.17 |
| 40G5c | 24131 | 0.89 |
| 12C | 28242 | 1.10 |
| 7221G20 | 9140 | 4.71 |
| F2B | 72 | Negative |
| hIgG1 | 74 | Negative |

### Example 12 Functional identification of humanized antibodies

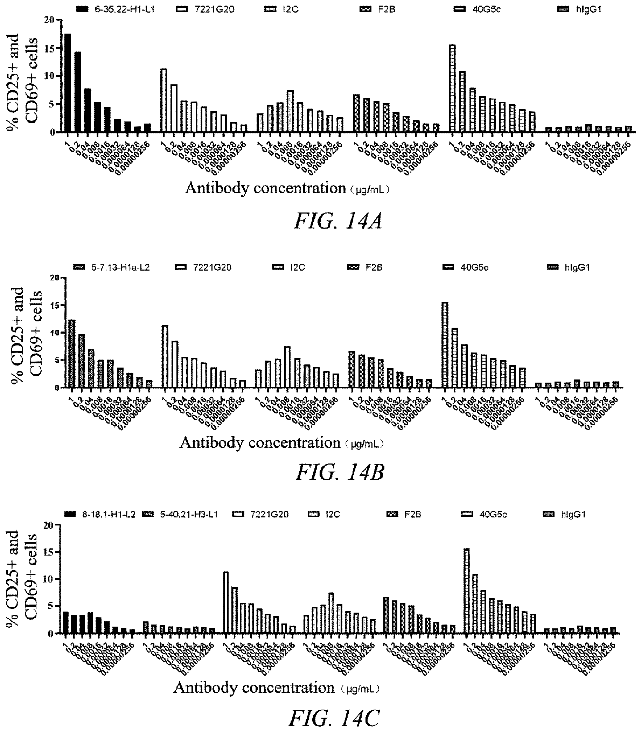

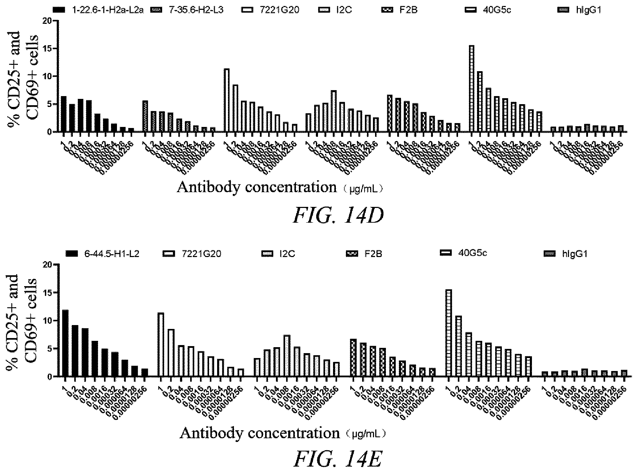

To detect the activation of the signaling pathway in CD3 cells by the humanized antibodies, the antibody to be detected was diluted with RPMI 1640 containing 10% FBS (initial concentration of 1*2 µg/mL, and gradient dilution at 1 : 5), and then added to a 96-well round bottom cell culture plate at 100 µl/well. Cryopreserved PBMC cells (purchased from AllCells Biotechnology (Shanghai) Co., Ltd.) was resuscitated, adjusted to the cell concentration of 1e6/mL, and then added to a cell culture plate added with an antibody at 100 µL/well. After incubation in an incubator at 37°C for 24 h, the plate was centrifuged and washed 1 time with PBS. A prepared FACS staining reagent (anti-CD25-PE, purchased from Biolegend, catalog number: 302602; anti-CD69-FITC, purchased from BD, catalog number: 555530) was added at 50 µL/well. The plate was incubated at 4°C for 45 min, and centrifuged and washed 3 times with a PBS buffer. FACS (FACS Canto, purchased from BD Company) was used for detection and result analysis. Data analysis was performed by software (CellQuest) to obtain the mean fluorescence density (MFI)of the cells. And then software (GraphPad PrismS) was used for analysis. The analysis results are as shown in FIGs. 14A-14E, indicating that all humanized antibodies have different degrees of activation on human PBMC.

## Claims

1. An antibody specifically binding to human CD3 or an antigen binding fragment thereof, wherein the antibody or the antigen binding fragment thereof comprises a heavy chain variable region and/or a light chain variable region, the heavy chain variable region comprises HCDR1-3, the light chain variable region comprises LCDR1-3, and the HCDR1-3 and/or the LCDR1-3 are selected from Table 10 or Table 18;
preferably, the HCDR1 comprises a sequence as shown in any one of SEQ ID NOs: 31-33, 44-46, 57-59, 70-72, 83-85, 96-98 and 109-111, or a sequence having at least 70% identity or at most 3 amino acid mutations compared thereto; and
the HCDR2 comprises a sequence as shown in any one of SEQ ID NOs: 34-36, 137, 47-49, 60-62, 73-75, 86-88, 99-101 and 112-114, or a sequence having at least 70% identity or at most 3 amino acid mutations compared thereto; and
the HCDR3 comprises a sequence as shown in any one of SEQ ID NOs: 37-38, 138-139, 50-51, 142-143, 63-64, 76-77, 89-90, 102-103 and 115-116, or a sequence having at least 70% identity or at most 3 amino acid mutations compared thereto; and/or
the LCDR1 comprises a sequence as shown in any one of SEQ ID NOs: 39-40, 140-141, 52-53, 65-66, 78-79, 91-92, 104-105 and 117-118, or a sequence having at least 70% identity or at most 3 amino acid mutations compared thereto; and
the LCDR2 comprises a sequence as shown in any one of SEQ ID NOs: 41-42, 54-55, 67-68, 80-81, 93-94, 106-107 and 119-120, or a sequence having at least 70% identity or at most 3 amino acid mutations compared thereto; and
the LCDR3 comprises a sequence as shown in any one of SEQ ID NOs: 43, 56, 69, 82, 95, 108 and 121, or a sequence having at least 70% identity or at most 3 amino acid mutations compared thereto;
and more preferably, the HCDR1-3 and/or the LCDR1-3 are determined according to the Kabat, Chothia or IMGT method.

2. The antibody or the antigen binding fragment thereof according to claim 1, wherein the heavy chain variable region comprises a sequence as shown in any one of SEQ ID NOs: 17, 19, 21, 23, 25, 27, 29, 124, 126, 128, 130, 132, 134 and 136, or a sequence having at least 70% identity or at most 15 amino acid mutations compared thereto; and/or
the light chain variable region comprises a sequence as shown in any one of SEQ ID NOs: 18, 20, 22, 24, 26, 28, 30, 123, 125, 127, 129, 131, 133 and 135, or a sequence having at least 70% identity or at most 15 amino acid mutations compared thereto.

3. The antibody or the antigen binding fragment thereof according to claim 1 or 2, wherein the at least 70% identity is at least 75%, at least 80%, at least 85%, at least 90%, at least 91%, at least 92%, at least 93%, at least 94%, at least 95%, at least 96%, at least 97%, at least 98%, at least 99% or 100% identity; and/or the at most 3 mutations are at most 3, 2, 1 or 0 mutations; and/or the at most 15 mutations are at most 15, 14, 13, 12, 11, 10, 9, 8, 7, 6, 5, 4, 3, 2, 1 or 0 mutations; preferably, the mutation is insertion, deletion or substitution; more preferably, the substitution is a conservative amino acid substitution; and most preferably, the mutation is a back mutation or a hotspot mutation.

4. The antibody or the antigen binding fragment thereof according to any one of claims 1-3, wherein
the heavy chain variable region of the antibody or the antigen binding fragment thereof has an amino acid sequence as shown in SEQ ID NO: 17, and the light chain variable region of the antibody or the antigen binding fragment thereof has an amino acid sequence as shown in SEQ ID NO: 18; or
the heavy chain variable region of the antibody or the antigen binding fragment thereof has an amino acid sequence as shown in SEQ ID NO: 19, and the light chain variable region of the antibody or the antigen binding fragment thereof has an amino acid sequence as shown in SEQ ID NO: 20; or
the heavy chain variable region of the antibody or the antigen binding fragment thereof has an amino acid sequence as shown in SEQ ID NO: 21, and the light chain variable region of the antibody or the antigen binding fragment thereof has an amino acid sequence as shown in SEQ ID NO: 22; or
the heavy chain variable region of the antibody or the antigen binding fragment thereof has an amino acid sequence as shown in SEQ ID NO: 23, and the light chain variable region of the antibody or the antigen binding fragment thereof has an amino acid sequence as shown in SEQ ID NO: 24; or
the heavy chain variable region of the antibody or the antigen binding fragment thereof has an amino acid sequence as shown in SEQ ID NO: 25, and the light chain variable region of the antibody or the antigen binding fragment thereof has an amino acid sequence as shown in SEQ ID NO: 26; or
the heavy chain variable region of the antibody or the antigen binding fragment thereof has an amino acid sequence as shown in SEQ ID NO: 27, and the light chain variable region of the antibody or the antigen binding fragment thereof has an amino acid sequence as shown in SEQ ID NO: 28; or
the heavy chain variable region of the antibody or the antigen binding fragment thereof has an amino acid sequence as shown in SEQ ID NO: 29, and the light chain variable region of the antibody or the antigen binding fragment thereof has an amino acid sequence as shown in SEQ ID NO: 30; or
the heavy chain variable region of the antibody or the antigen binding fragment thereof has an amino acid sequence as shown in SEQ ID NO: 124, and the light chain variable region of the antibody or the antigen binding fragment thereof has an amino acid sequence as shown in SEQ ID NO: 123; or
the heavy chain variable region of the antibody or the antigen binding fragment thereof has an amino acid sequence as shown in SEQ ID NO: 126, and the light chain variable region of the antibody or the antigen binding fragment thereof has an amino acid sequence as shown in SEQ ID NO: 125; or
the heavy chain variable region of the antibody or the antigen binding fragment thereof has an amino acid sequence as shown in SEQ ID NO: 128, and the light chain variable region of the antibody or the antigen binding fragment thereof has an amino acid sequence as shown in SEQ ID NO: 127; or
the heavy chain variable region of the antibody or the antigen binding fragment thereof has an amino acid sequence as shown in SEQ ID NO: 130, and the light chain variable region of the antibody or the antigen binding fragment thereof has an amino acid sequence as shown in SEQ ID NO: 129; or
the heavy chain variable region of the antibody or the antigen binding fragment thereof has an amino acid sequence as shown in SEQ ID NO: 132, and the light chain variable region of the antibody or the antigen binding fragment thereof has an amino acid sequence as shown in SEQ ID NO: 131; or
the heavy chain variable region of the antibody or the antigen binding fragment thereof has an amino acid sequence as shown in SEQ ID NO: 134, and the light chain variable region of the antibody or the antigen binding fragment thereof has an amino acid sequence as shown in SEQ ID NO: 133; or
the heavy chain variable region of the antibody or the antigen binding fragment thereof has an amino acid sequence as shown in SEQ ID NO: 136, and the light chain variable region of the antibody or the antigen binding fragment thereof has an amino acid sequence as shown in SEQ ID NO: 135.

5. The antibody or the antigen binding fragment thereof according to any one of claims 1-4, wherein the antibody or the antigen binding fragment thereof further comprises a heavy chain constant region and/or a light chain constant region;
optionally, the heavy chain constant region is selected from IgG, such as IgG1, IgG2, IgG3 or IgG4; preferably, the heavy chain constant region is selected from human IgG, such as human IgG1, human IgG2, human IgG3 or human IgG4; more preferably, the heavy chain constant region is selected from SEQ ID NO: 13; and/or
the light chain constant region is selected from a κ chain or a λ chain;
and preferably, the light chain constant region is selected from SEQ ID NO: 14 and SEQ ID NO: 122.

6. The antibody or the antigen binding fragment thereof according to any one of claims 1-5, wherein the antibody or the antigen binding fragment thereof is selected from: a monoclonal antibody, a polyclonal antibody, a natural antibody, an engineered antibody, a mono-specific antibody, a multi-specific antibody (for example, a bispecific antibody), a monovalent antibody, a multivalent antibody, an intact antibody, a fragment of an intact antibody, a naked antibody, a conjugated antibody, a chimeric antibody, a humanized antibody, a fully human antibody, Fab, Fab', Fab'-SH, F(ab')₂, Fd, Fv, scFv, a diabody and a single domain antibody.

7. The antibody or the antigen binding fragment thereof according to any one of claims 1-6, wherein the antibody or the antigen binding fragment thereof is conjugated with a therapeutic agent or a tracer; preferably, the therapeutic agent is selected from: a radioactive isotope, a chemotherapeutic drug and an immunomodulator; and/or the tracer is selected from: a radiological contrast agent, a paramagnetic ion, a metal, a fluorescent tag, a chemiluminescence tag, an ultrasound contrast agent and a photosensitizer.

8. The antibody or the antigen binding fragment thereof according to any one of claims 1-7, wherein the antibody or the antigen binding fragment thereof also binds to monkey CD3.

9. The antibody or the antigen binding fragment thereof according to any one of claims 1-8, wherein the CD3 is selected from a CD3ε subunit and a dimer comprising a CD3ε subunit, such as a CD3ε/δ dimer.

10. A multi-specific antigen binding molecule, wherein the multi-specific antigen binding molecule comprises at least a first antigen binding module and a second antigen binding module, the first antigen binding module comprises the antibody or the antigen binding fragment thereof according to any one of claims 1-9, and/or the second antigen binding module binds to other targets different from the first antigen binding module or binds to different epitopes of the same target; preferably, the other targets are selected from a tumor specific antigen (TSA) and a tumor associated antigen (TAA); and optionally, the multi-specific antigen binding molecule is a bispecific T cell engager (BiTE).

11. An isolated nucleic acid molecule, wherein the nucleic acid molecule encodes the antibody or the antigen binding fragment thereof according to any one of claims 1-9, or the multi-specific antigen binding molecule according to claim 10; and optionally, the nucleic acid molecule also encodes a chimeric antigen receptor.

12. A vector, comprising the nucleic acid molecule according to claim 11.

13. A cell, comprising the vector according to claim 12.

14. An immune effector cell, expressing a chimeric antigen receptor, and/or expressing the antibody or the antigen binding fragment thereof according to any one of claims 1-9, or the multi-specific antigen binding molecule according to claim 10;
preferably, the immune effector cell is selected from: a T cell, a natural killer cell (NK cell), a natural killer T cell (NKT cell), a monocyte, a macrophage, a dendritic cell and a mast cell; more preferably, the T cell is selected from: a cytotoxic T cell, a regulatory T cell and a helper T cell; and/or
preferably, the immune effector cell is an autologous immune effector cell or an allogeneic immune effector cell.

15. A method for preparing the antibody or the antigen binding fragment thereof according to any one of claims 1-9, or the multi-specific antigen binding molecule according to claim 10, wherein the method comprises: (1) culturing the cell according to claim 13; and/or (2) isolating the antibody or the antigen binding fragment thereof, or the multi-specific antigen binding molecule expressed by the cell.

16. A method for preparing the immune effector cell according to claim 14, wherein the method comprises: (1) introducing into a naive immune effector cell a. a nucleic acid molecule encoding the chimeric antigen receptor and b. a nucleic acid molecule encoding the antibody or the antigen binding fragment thereof according to any one of claims 1-9, or the multi-specific antigen binding molecule according to claim 10; and/or (2) making the immune effector cell into which the above-mentioned nucleic acid molecules are introduced express the chimeric antigen receptor, and the antibody or the antigen binding fragment thereof according to any one of claims 1-9, or the multi-specific antigen binding molecule according to claim 10.

17. A pharmaceutical composition, wherein the pharmaceutical composition comprises the antibody or the antigen binding fragment thereof according to any one of claims 1-9, the multi-specific antigen binding molecule according to claim 10, the nucleic acid molecule according to claim 11, the vector according to claim 12, the cell according to claim 13, the immune effector cell according to claim 14 or a product prepared according to the method according to claim 15 or 16; and preferably, the composition further comprises a pharmaceutically acceptable carrier, a diluent or an auxiliary agent.

18. Use of the antibody or the antigen binding fragment thereof according to any one of claims 1-9, the multi-specific antigen binding molecule according to claim 10, the nucleic acid molecule according to claim 11, the vector according to claim 12, the cell according to claim 13, the immune effector cell according to claim 14, a product prepared according to the method according to claim 15 or 16, or the pharmaceutical composition according to claim 17 in the preparation of a drug for treating a tumor or a cancer;
preferably, the tumor or the cancer is selected from a hematological tumor and a solid tumor; more preferably, the hematological tumor is selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma; and/or the solid tumor is selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer and liver cancer.

19. A method for treating a tumor or a cancer, wherein the method comprises administering to a subject an effective amount of a drug, and the drug comprises the antibody or the antigen binding fragment thereof according to any one of claims 1-9, the multi-specific antigen binding molecule according to claim 10, the nucleic acid molecule according to claim 11, the vector according to claim 12, the cell according to claim 13, the immune effector cell according to claim 14, a product prepared according to the method according to claim 15 or 16, or the pharmaceutical composition according to claim 17;
preferably, the tumor or the cancer is selected from a hematological tumor and a solid tumor; more preferably, the hematological tumor is selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma; and/or the solid tumor is selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer and liver cancer.

20. The antibody or the antigen binding fragment thereof according to any one of claims 1-9, the multi-specific antigen binding molecule according to claim 10, the nucleic acid molecule according to claim 11, the vector according to claim 12, the cell according to claim 13, the immune effector cell according to claim 14, a product prepared according to the method according to claim 15 or 16, or the pharmaceutical composition according to claim 17, for use in the treatment of a tumor or a cancer;
preferably, the tumor or the cancer is selected from a hematological tumor and a solid tumor; more preferably, the hematological tumor is selected from: acute myelogenous leukemia (AML), chronic myelogenous leukemia (CML), CML in blastic phase, myelodysplastic syndrome (MDS), B-acute lymphocytic leukemia (B-ALL), T-acute lymphocytic leukemia (TALL), chronic lymphocytic leukemia (CLL), Richter syndrome, hairy cell leukemia (HCL), blastic plasmacytoid dendritic cell neoplasm (BPDCN), non-Hodgkin lymphoma (NHL) including mantle cell lymphoma (MCL) and small lymphocytic lymphoma (SLL), Hodgkin lymphoma, systemic mastocytosis and Burkitt lymphoma; and/or the solid tumor is selected from: ovarian cancer, pancreatic cancer, lung cancer, gastric cancer, esophageal cancer, gastroesophageal cancer, colorectal cancer, prostate cancer, kidney cancer, bladder cancer, glioma, breast cancer and liver cancer.
